# EUROPEAN PATENT APPLICATION

(11) **EP 4 311 834 A1**
(43) Date of publication of application: **31.01.2024**
(21) Application number: 22187930.7
(22) Date of filing: 29.07.2022
(51) Int. Cl.: C07K 16/18, C07K 16/08, A61P 35/00

(54) **PEPTIDES AND COMBINATIONS OF PEPTIDES FOR USE IN IMMUNOTHERAPY AGAINST ROPHARYNGEAL SQUAMOUS CELL CARCINOMA (OPSCC) AND OTHER CANCERS**

(71) Applicant: Eberhard Karls Universität Tübingen, Medizinische Fakultät, 72074 Tübingen (DE)
(72) Inventor: Laban, Simon, 89081 Ulm (DE); Mühlenbruch, Lena, 72072 Tübingen (DE); Walz, Juliane, 72074 Tübingen (DE); Rammensee, Hans-Georg, 72070 Unterjesingen (DE); Hoffmann, Thomas, 89081 Ulm (DE)
(74) Representative: Witte, Weller & Partner Patentanwälte mbB

(57) **Abstract**

The present invention relates to peptides, proteins, nucleic acids and cells for use in immunotherapeutic methods. In particular, the present invention relates to the immunotherapy of cancer, in particular of oropharyngeal squamous cell carcinoma (OPSCC). The present invention furthermore relates to tumor-associated T-cell peptide epitopes that can for example serve as active pharmaceutical ingredients of vaccine compositions that stimulate anti-tumor immune responses, or to stimulate T cells ex *vivo* and transfer into patients. Peptides bound to molecules of the major histocompatibility complex (MHC), or peptides as such, can also be targets of antibodies, soluble T-cell receptors, and other binding molecules.

## Description

The present invention relates to peptides, proteins, nucleic acids and cells for use in immunotherapeutic methods. In particular, the present invention relates to the immunotherapy of cancer, in particular of oropharyngeal squamous cell carcinoma (OPSCC). The present invention furthermore relates to tumor-associated T-cell peptide epitopes that can for example serve as active pharmaceutical ingredients of vaccine compositions that stimulate anti-tumor immune responses, or to stimulate T cells *ex vivo* and transfer into patients. Peptides bound to molecules of the major histocompatibility complex (MHC), or peptides as such, can also be targets of antibodies, soluble T-cell receptors, and other binding molecules.

The present invention relates to several novel peptide sequences and their variants that can be used in vaccine compositions for eliciting anti-tumor immune responses, in particular against oropharyngeal squamous cell carcinoma (OPSCC), or as targets for the development of pharmaceutically/immunologically active compounds and cells.

### FIELD OF THE INVENTION

The present invention relates to the field of molecular biology or medicine, more particular to the field of molecular immunology.

### BACKGROUND OF THE INVENTION

In 2015, about 90.5 million people worldwide had cancer. In 2019, annual cancer cases grew to 23.6 million people and 10 million deaths worldwide, representing over the previous decade increases of 26% and 21%, respectively. Cancer is the second leading cause of death globally, accounting for an estimated 9.6 million deaths, or one in six deaths, in 2018. One in 5 men and one in 6 women worldwide develop cancer during their lifetime, and one in 8 men and one in 11 women die from the disease. Worldwide, the total number of people who are alive within 5 years of a cancer diagnosis, called the 5-year prevalence, is estimated to be 43.8 million.

The most common types of cancer in males are lung cancer, prostate cancer, colorectal cancer, and stomach cancer. In females, the most common types are breast cancer, colorectal cancer, lung cancer, and cervical cancer. If skin cancer other than melanoma were included in total new cancer cases each year, it would account for around 40% of cases. In children, acute lymphoblastic leukemia and brain tumors are most common, except in Africa, where non-Hodgkin lymphoma occurs more often. In 2012, about 165,000 children under 15 years of age were diagnosed with cancer. The risk of cancer increases significantly with age, and many cancers occur more commonly in developed countries. Rates are increasing as more people live to an old age and as lifestyle changes occur in the developing world.

Oropharyngeal squamous cell carcinoma (OPSCC), also known as oropharyngeal cancer (OPC) and tonsil cancer, is a disease in which abnormal cells with the potential to both grow locally and spread to other parts of the body are found in the oral cavity, in the tissue of the part of the throat (oropharynx) that includes the base of the tongue, the tonsils, the soft palate, and the walls of the pharynx.

OPSCC is diagnosed in 93,000 patients worldwide per year and 51,000 annual deaths can be attributed to this disease. In OPSCC, human papillomavirus induced (HPV-positive) cancers and non-virally associated, primarily tobacco and alcohol associated (HPV-negative) cancers must be discriminated. For different types of curative treatment, a survival advantage for HPV-positive OPSCC has been confirmed. As a result, the latest classification of the American Joint Committee on Cancer (AJCC) cancer staging manual version 8 discriminates between HPV-positive and HPV-negative cancers based on the surrogate marker p16.

Oropharyngeal cancer is diagnosed by biopsy of observed abnormal tissue in the throat. OPSCC is staged according to the appearance of the abnormal cells on the biopsy coupled with the dimensions and the extent of the abnormal cells found.

Current treatment of OPSCC is with surgery, chemotherapy, or radiation therapy, or some combination of those treatments. Despite their widespread use, such treatment is frequently ineffective, but nonetheless can substantially reduce the patient's quality of life. In particular, salvage surgery often has to be extensive and is mutilating for the patient.

In recent years, T cell-based immunotherapy approaches such as checkpoint inhibitors, CAR T cells, adoptive T cell transfer and vaccination strategies have become increasingly important for the treatment of solid tumors. The identification of target structures on the surface of tumor cells forms the basis for the development of these antigen-specific therapeutic strategies. Suitable target structures have the characteristics of being naturally, highly abundant and tumor-exclusive presented on cell surfaces and recognized by the patient's immune system. They are represented by peptides presented by HLA class I and II molecules on the surface of tumor cells.

Immunotherapeutic strategies provide promising tools to develop new treatment options for OPSCC patients. This is particularly true for the highly T-cell infiltrated HPV (human papillomavirus)-positive OPSCCs, whose proportion within the entity of OPSCCs is currently growing significantly.

Immunotherapy targeting the PD1/PD-L1 axis has become a central column of treatment in recurrent and metastatic disease and is currently studied intensively in locoregionally advanced disease. In recurrent and metastatic disease, the response rates of anti-PD1 antibodies lie below 20%. Because the success of PD1/PD-L1 antibodies relies on the presence of pre-existing cancer-antigen specific immunity, vaccination against cancer antigens may improve efficacy of such treatments. However, it is currently unclear which antigens should be targeted. Immune responses to viral antigens in HPV-positive disease and immune responses to other cancer antigens including mutation-associated antigens, so called neoantigens, have previously been described. The respective significance of the different types of cancer antigens for immunotherapy is currently unclear.

To date, very few target antigens have been described for immunotherapeutic treatment of OPSCCs. OPSCCs exhibit elevated expression levels of cancer/testis antigens, which is why these have been proposed as potential target antigens. The use of a T-cell epitope derived from the cancer/testis antigen IMP3 (U3 small nucleolar ribonucle-oprotein protein) already achieved promising results in a clinical vaccination trial in terms of improved prognosis for OPSCC patients.

A summary on the currently known strategies to treat head and neck squamous cell carcinomas (HNSCC) including OPSCC by immunotherapy can be found in Witzleben et al., HNSCC: Tumour Antigens and Their Targeting by Immunotherapy. Cells 9, 2103, 20-30 (2020).

None of the immunotherapy approaches against OPSCC ever reached broad clinical application and efficacy, let alone approval. This may have its reason in the relative unsuitability of the known peptides to elicit OPSCC-specific immune responses in the patients.

It is, therefore, an object underlying the invention to provide tumor-associated T-cell peptide epitopes, which can be used to develop improved medicaments and methods for the diagnosis, prophylaxis and treatment of cancer, in particular of OPSCC. In particular, a pharmaceutical composition, such as a peptide-based vaccine should be provided, which can be used for the diagnosis and/or prevention and/or treatment of OPSCC.

### SUMMARY OF THE INVENTION

The present invention provided a peptide comprising an amino acid sequence selected from the group consisting of SEQ ID NO: 1 to SEQ ID NO: 62, and variant sequences thereof which are at least 88% homologous to SEQ ID NO: 1 to SEQ ID NO: 62, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

The inventors have realized that the strategy used in the art for identifying tumor antigens on the basis of gene expression analyzes and *in silico* prediction of the resulting HLA ligands has significant drawbacks. This approach is decisively hindered by the distorted correlation between gene expression and HLA-restricted antigen presentation. I.e. it cannot be predicted from gene expression whether or not an antigen is actually presented on the cell surface of tumor cells.

The inventors, therefore, applied a novel strategy. The inventors have based the selection of widely applicable peptides on the frequency of their presentation within an examined patient cohort. The inventors were, therefore, able to identify the peptides according to the invention by direct analysis of the HLA ligands of OPSCC samples.

As a result of this approach, the inventors were able to identify 14 different OPSCC-specific MHC/HLA class II peptides comprising amino acid sequences of SEQ ID NO: 1 to SEQ ID NO: 14. Such MHC/HLA class II peptides can be used in any OPSCC patient independent of its MHC/HLA allotype. The inventors further identified 48 different OPSCC-specific MHC/HLA class I peptides comprising the amino acid sequences of SEQ ID NO: 15 to SEQ ID NO: 62. These are the MHC/HLA class I presented peptides of HLA allotypes A*02:01 (SEQ ID NO: 15 to SEQ ID NO: 24), A*01:01 (SEQ ID NO: 25 to SEQ ID NO: 31), A*03:01 (SEQ ID NO: 32), A*24:02 (SEQ ID NO: 33 to SEQ ID NO: 35), A*11:01 (SEQ ID NO: 36), B*07:02 (SEQ ID NO: 37 to SEQ ID NO: 38), B*51:01 (SEQ ID NO: 39 to SEQ ID NO: 41), B*08:01 (SEQ ID NO: 42 to SEQ ID NO: 45), B*44:02 (SEQ ID NO: 46 to SEQ ID NO: 48), B*40:01 (SEQ ID NO: 49 to SEQ ID NO: 53), C*07:01 (SEQ ID NO: 54 to SEQ ID NO: 55), C*07:02 (SEQ ID NO: 56), C*04:01 (SEQ ID NO: 57 to SEQ ID NO: 58), C*03:04 (SEQ ID NO: 59 to SEQ ID NO: 61) und C*06:02 (SEQ ID NO: 62).

All selected peptides were identified with high frequency and exclusively in the cohort immunopeptidome, whereas they were never identified in the healthy controls.

The following table 1 shows the MHC/HLA class II peptides according to the invention.

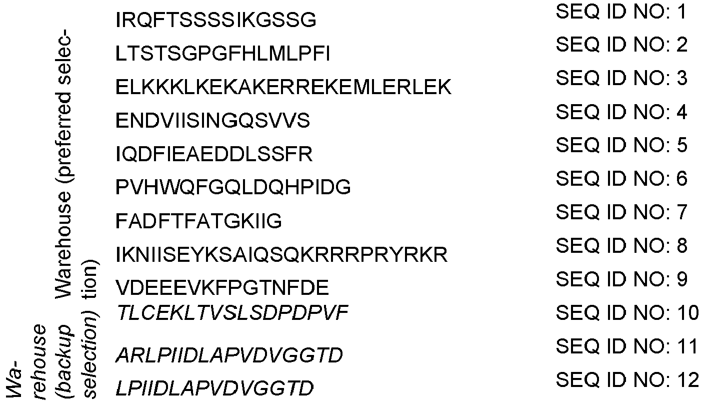

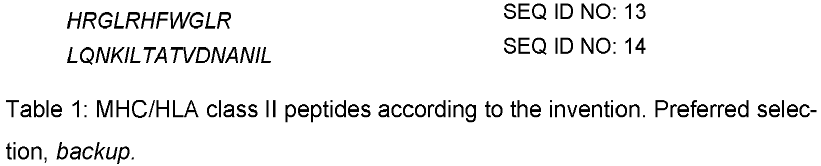

The following table 2 shows the MHC/HLA class I peptides according to the invention.

**Table 2: MHC/HLA class I peptides according to the invention. Preferred selection; backup selection.**

| **Allotype** | passed QC | | | | | | | | | |
|---|---|---|---|---|---|---|---|---|---|---|
| **A^{∗}02:01** | GVLENIFGV (SEQ ID NO: 15) | KLAEISLGV (SEQ ID NO: 16) | RLDDLKMTV (SEQ ID NO: 17) | GLWEDGRSTLL (SEQ ID NO: 18) | ALLGSAFQL (SEQ ID NO: 19) | RLLEGEDAHL (SEQ ID NO: 20) | ALTDIVSQV (SEQ ID NO: 21) | *RLLDSVSRL* (SEQ ID NO: 22) | *VLFPNLKTV* (SEQ ID NO: 23) | *YLEEFITNI* (SEQ ID NO: 24) |
| **A^{∗}01:01** | RTEFNLNQY (SEQ ID NO: 25 | EMEAQNQEY (SEQ ID NO: 26) | RTDIARTEY (SEQ ID NO: 27) | YSELASHVVSY (SEQ ID NO: 28) | *EMEQQNQEY* (SEQ ID NO: 29) | *VLDSHIHAY* (SEQ ID NO: 30) | *GTEDELDKY* (SEQ ID NO: 31) | | | |
| **A^{∗}03:01** | *RLRGGSPLNTGK* (SEQ ID NO: 32) | | | | | | | | | |
| **A^{∗}24:02** | KYMYFTVVM (SEQ ID NO: 33) | NWPSRPYLF (SEQ ID NO: 34) | *GYIDNVTLI* (SEQ ID NO: 35) | | | | | | | |
| **A^{∗}11:01** | *AVGPPTTVR* (SEQ ID NO: 36) | | | | | | | | | |
| **B^{∗}07:02** | *APAAWLRSAA* (SEQ ID NO: 37) | *APKAPGSPW* (SEQ ID NO: 38) | | | | | | | | |
| **B^{∗}51:01** | DATETTITI (SEQ ID NO: 39) | DQYKFLAV (SEQ ID NO: 40) | VALPVYLLI (SEQ ID NO: 41) | | | | | | | |
| **B^{∗}08:01** | HGTIKNQL (SEQ ID NO: 42) | VAAPRWVL (SEQ ID NO: 43) | *EIKFKVETL* (SEQ ID NO: 44 | *SASPKEKYSL* (SEQ ID NO: 45) | | | | | | |
| **B^{∗}44:02** | EETNPKGSGW (SEQ ID NO: 46) | *IEDDPKMMW* (SEQ ID NO: 47) | *SEAESRIFW* (SEQ ID NO: 48 | | | | | | | |
| **B^{∗}40:01** | REVVDPEVFF (SEQ ID NO: 49) | SEPNDVFFKL (SEQ ID NO: 50) | *SEVVDVAKL* (SEQ ID NO: 51) | *VEDSALLMQTL* (SEQ ID NO: 52) | *MEAPAQLLFLL* (SEQ ID NO: 53) | | | | | |
| **C^{∗}07:01** | YTFRYPLSL (SEQ ID NO: 54) | ARLAFVIVF (SEQ ID NO: 55) | | | | | | | | |
| **C^{∗}07:02** | *RQLVFNEI* (SEQ ID NO: 56) | | | | | | | | | |
| **C^{∗}04:01** | SPEDGIHEL (SEQ ID NO: 57) | VFDLVELEVL (SEQ ID NO: 58) | | | | | | | | |
| **C^{∗}03:04** | YEIDDVERL (SEQ ID NO: 59) | SAVKEGTAM (SEQ ID NO: 60) | YEEAMKEVL(SEQ ID NO: 61) | | | | | | | |
| **C^{∗}06:02** | ARLIPIIVL (SEQ ID NO: 62) | | | | | | | | | |

In the event of discrepancies between the sequences specified in tables 1 and 2 and those specified in the sequence listing, the information in the tables takes precedence and applies.

The term "peptide" is used herein to designate a series of amino acid residues, connected one to the other typically by peptide bonds between the alpha-amino and carbonyl groups of the adjacent amino acids. The peptides are preferably between 7 and 12 amino acids in length, further preferably between 8 and 11, but can be as long as 5, 6, 7, 8, 9, 10, 11, 12, 13, 14, 15, 16, 17, 18, 19, 20, 21 or longer.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "peptide" shall also include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 15 amino acids in length.

The term "peptide" shall also include "polypeptide". The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an MHC molecule in substantially the same way as a peptide consisting of the given amino acid sequence in consisting of SEQ ID NO: 1 to SEQ ID NO: 62. For example, a peptide may be modified so that it at least maintains, if not improves, the ability to interact with and bind to the binding groove of a suitable MHC molecule and in that way, it at least maintains, if not improves, the ability to bind to the TCR of activated T cells.

The original (unmodified) peptides as disclosed herein can be modified by the substitution of one or more residues at different, possibly selective, sites within the peptide chain, if not otherwise stated. Preferably those substitutions are located at the end of the amino acid chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions". Conservative substitutions are herein defined as exchanges within one of the following five groups: Group 1-small aliphatic, nonpolar or slightly polar residues (Ala, Ser, Thr, Pro, GIY); Group 2-polar, negatively charged residues and their amides (Asp, Asn, Glu, Gln); Group 3-polar, positively charged residues (His, Arg, Lys); Group 4-large, aliphatic, nonpolar residues (Met, Leu, Ile, Val, Cys); and Group 5-large, aromatic residues (Phe, Tyr, Trp). Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such "radical" substitutions cannot, however, be dismissed as potentially ineffective since chemical effects are not totally predictable and radical substitutions might well give rise to serendipitous effects not otherwise predictable from simple chemical principles. Of course, such substitutions may involve structures other than the common L-amino acids. Thus, D-amino acids might be substituted for the L-amino acids commonly found in the antigenic peptides of the invention and yet still be encompassed by the disclosure herein. In addition, non-standard amino acids (i.e., other than the common naturally occurring proteinogenic amino acids) may also be used for substitution purposes to produce immunogens and immunogenic polypeptides according to the present invention.

If substitutions at more than one position are found to result in a peptide with substantially equivalent or greater antigenic activity as defined below, then combinations of those substitutions will be tested to determine if the combined substitutions result in additive or synergistic effects on the antigenicity of the peptide. At most, no more than 4 positions within the peptide would be simultaneously substituted.

The amino acid residues that do not substantially contribute to interactions with the T cell receptor can be modified by replacement with other amino acids whose incorporation do not substantially affect T cell reactivity and does not eliminate binding to the relevant MHC.

Longer (elongated) peptides may also be suitable. It is possible that MHC class I epitopes, although usually between 8 and 11 amino acids long, are generated by peptide processing from longer peptides or proteins that include the actual epitope. It is preferred that the residues that flank the actual epitope are residues that do not substantially affect proteolytic cleavage necessary to expose the actual epitope during processing.

The peptides of the invention can be elongated by up to four amino acids, that is 1, 2, 3 or 4 amino acids can be added to either end in any combination between 4:0 and 0:4. Combinations of the elongations according to the invention can be found in Table 2.

**Table 3: Combinations of the elongations of peptides of the invention**

| C-terminus | N-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

| N-terminus | C-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

The amino acids for the elongation/extension can be from the peptides of the original sequence of the protein or any other amino acid(s). The elongation can be used to enhance the stability or solubility of the peptides.

Thus, the epitopes of the present invention may be identical to naturally occurring tumor-associated or tumor-specific epitopes or may include epitopes that differ by no more than four residues from the reference peptide, as long as they have substantially identical antigenic activity.

In an alternative embodiment, the peptide is elongated on either or both sides by more than 4 amino acids, preferably to a total length of up to 30 amino acids. This may lead to MHC class II binding peptides. Binding to MHC class II can be tested by methods known in the art.

Accordingly, the present invention provides peptides and variants of MHC class I epitopes, wherein the peptide or variant has an overall length of between 8 and 100, preferably between 8 and 30, and most preferred between 8 and 14, namely 8, 9, 10, 11, 12, 13, 14 amino acids, in case of the elongated class II binding peptides the length can also be 15, 16, 17, 18, 19, 20, 21 or 22 or 23 amino acids.

Of course, the peptide or variant according to the present invention will have the ability to bind to a molecule of the human major histocompatibility complex (MHC/HLA) class I or II. Binding of a peptide or a variant to an MHC complex may be tested by methods known in the art.

Preferably, when the T cells specific for a peptide according to the present invention are tested against the substituted peptides, the peptide concentration at which the substituted peptides achieve half the maximal increase in lysis relative to background is no more than about 1 mM, preferably no more than about 1 µM, more preferably no more than about 1 nM, and still more preferably no more than about 100 pM, and most preferably no more than about 10 pM. It is also preferred that the substituted peptide be recognized by T cells from more than one individual, at least two, and more preferably three individuals.

A person skilled in the art will be able to assess, whether T cells induced by a variant of a specific peptide will be able to cross-react with the peptide itself (Appay et al., 2006; Colombetti et al., Eur. J. Immunol. 36: 1805-1814 (2006); Fong et al., Proc. Natl. Acad. Sci. U.S.A. 98: 8809-8814 (2001); Zaremba et al., Cancer Res. 57: 4570-4577 (1997)).

These T cells can subsequently cross-react with cells and kill cells that express a polypeptide that contains the natural amino acid sequence of the cognate peptide as defined in the aspects of the invention. As can be derived from the scientific literature and databases (Rammensee et al., Immunogenetics 50: 213-219 (1999); Godkin et al., Int. Immunol 9: 905-911 (1997)), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. Thus, one skilled in the art would be able to modify the amino acid sequences set forth in SEQ ID NO: 1 to SEQ ID NO: 62, by maintaining the known anchor residues, and would be able to determine whether such variants maintain the ability to bind MHC class I or II molecules. The variants of the present invention retain the ability to bind to the TCR of activated T cells, which can subsequently cross-react with and kill cells that express a polypeptide containing the natural amino acid sequence of the cognate peptide as defined in the aspects of the invention.

In the present invention, the term "homologous" refers to the degree of identity between sequences of two amino acid sequences, i.e. peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each base or amino acid in the Reference Sequence that does not have a corresponding aligned base or amino acid in the Compared Sequence and
(ii) each gap in the Reference Sequence and
(iii) each aligned base or amino acid in the Reference Sequence that is different from an aligned base or amino acid in the Compared Sequence, constitutes a difference and
(iv) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

According to the invention "full-length polypeptide" refers to the source proteins from which the peptides are derived. Full-length polypeptides are also referred to as the source genes/proteins from which the peptides are derived. The source proteins or full-length polypeptides may or may not be highly over-expressed in cancer compared with normal tissues. "Normal tissues" in relation to this invention shall mean either healthy peripheral blood mononuclear cells (PBMC) cells or other normal tissue cells, demonstrating a high degree of tumor association of the source genes. Moreover, the peptides themselves are presented on tumor tissue. "Tumor tissue" in relation to this invention shall mean a sample from a patient suffering from cancer, such oropharyngeal squamous cell carcinoma (OPSCC). Exemplary "full-length polypeptides" or source proteins are indicated in table 9, column "Source protein" or "Protein name", respectively.

In an embodiment of the invention the peptide is isolated. The term "isolated" means that the material is removed from its original environment (e.g., the natural environment, if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The peptides and/or nucleotides disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from a cDNA library have been conventionally purified to electrophoretic homogeneity. Purification of starting material or natural material to at least one order of magnitude, preferably two or three orders, and more preferably four or five orders of magnitude is expressly contemplated. Furthermore, a claimed polypeptide which has a purity of preferably 99.999%, or at least 99.99% or 99.9%; and even desirably 99% by weight or greater is expressly encompassed.

The peptides and nucleic acids according to the invention may be in "enriched form". As used herein, the term "enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

Against this background, another subject-matter of the invention is a pharmaceutical composition for the diagnosis and/or prevention and/or treatment of oropharyngeal squamous cell carcinoma (OPSCC) comprising at least one peptide which binds to class II molecules of the major histocompatibility complex (MHC class II molecules) and/or induces T cells cross-reacting with said peptide, and a pharmaceutically acceptable carrier, wherein said at least one peptide comprises an amino acid sequence which is selected from the group consisting of: SEQ ID NO: 1 to SEQ ID NO: 14 and variant sequences thereof which are at least 88% homologous to SEQ ID NO: 1 to SEQ ID NO: 14, wherein said peptide is not a full-length polypeptide.

Such pharmaceutical composition comprises at least one of the MHC class II peptides according to the invention. It is, therefore, independent from the allotype of the patient.

The features, advantages and embodiments of the peptide(s) according to the invention apply to the pharmaceutical composition according to the invention *mutatis mutandis.*

A "pharmaceutical composition" is a composition suitable for administration to a human being in a medical setting. Preferably, a pharmaceutical composition is sterile and produced according to GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH₂ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

Preferably, the pharmaceutical composition of the present invention is an immunotherapeutic such as a vaccine. It may be administered directly into the patient, into the affected organ or systemically i.d., i.m., s.c., i.p. and i.v., or applied *ex vivo* to cells derived from the patient or a human cell line which are subsequently administered to the patient, or used *in vitro* to select a subpopulation of immune cells derived from the patient, which are then re-administered to the patient. If the nucleic acid is administered to cells *in vitro,* it may be useful for the cells to be transfected so as to co-express immune-stimulating cytokines, such as interleukin-2.

The peptide(s) according to the invention may be substantially pure. The peptide(s) may also be combined with an immune-stimulating adjuvant, such as the TLR1/2 ligand XS15. Preliminary work has shown that XS15 can induce a strong CD8⁺ and Th1CD4⁺ T-cell response *in vivo* after subcutaneous injection in healthy donors and individual tumor patients for viral, mutated and unmutated peptides in a water-oil emulsion. The peptide(s) may also be used in combination with immune-stimulatory cytokines, or be administered with a suitable delivery system, for example liposomes. The peptide(s) may also be conjugated to a suitable carrier such as keyhole limpet haemocyanin (KLH) or mannan (see WO 95/18145 and (Longenecker et al., Ann. N.Y. Acad. Sci. 690:276-291 (1993)). The peptide(s) may also be tagged, may be a fusion protein, or may be a hybrid molecule. The peptides whose sequence is given in the present invention are expected to stimulate CD4 or CD8 T cells. However, stimulation of CD8 T cells is more efficient in the presence of help provided by CD4 T-helper cells. Thus, for MHC Class I epitopes that stimulate CD8 T cells the fusion partner or sections of a hybrid molecule suitably provide epitopes which stimulate CD4-positive T cells. CD4- and CD8-stimulating epitopes are well known in the art and include those identified in the present invention.

According to the invention, "at least one" means 1, 2, 3, 4, 5, 6,7, 8, 9, 10, 11, 12, 13, 14, 15, 16, 17, 18, 19, 20, 21, etc. This ensures coverage of a very high percentage of the world population.

The pharmaceutical composition according to the invention may comprise, as the only active ingredient, the peptide(s) according to the invention. However, in an alternative aspect, it may comprise an additional active ingredient.

The problem underlying the invention is herewith completely solved.

In an embodiment of the invention the pharmaceutical composition comprises at least 2, preferably at least 3, further preferably at least 4, further preferably at least 5, further preferably at least 6, further preferably at least 7, further preferably at least 8, and highly preferably at least 9 different peptides, each peptide comprising an amino acid sequence which is selected from the group consisting of: SEQ ID NO: 1 to SEQ ID NO: 14 and variant sequences thereof which are at least 88% homologous to SEQ ID NO: 1 to SEQ ID NO: 14, wherein said peptide is not a full-length polypeptide.

This measure has the advantage that, by increasing the number of different MHC class II peptides in the pharmaceutical an increasingly increasing number of individuals will be immunized, regardless of their specific allotype. With 9 different peptides, all population members are covered.

In another embodiment of the pharmaceutical composition according to the invention comprises different peptides comprising the following amino acid sequences:
IRQFTSSSSIKGSSG (SEQ ID NO: 1)
LTSTSGPGFHLMLPFI (SEQ ID NO: 2)
ELKKKLKEKAKERREKEMLERLEK (SEQ ID NO: 3)
ENDVIISINGQSVVS (SEQ ID NO: 4)
IQDFIEAEDDLSSFR (SEQ ID NO: 5)
PVHWQFGQLDQHPIDG (SEQ ID NO: 6)
FADFTFATGKIIG (SEQ ID NO: 7)
IKNIISEYKSAIQSQKRRRPRYRKR (SEQ ID NO: 8)
VDEEEVKFPGTNFDE (SEQ ID NO: 9).

In still another embodiment of the invention said pharmaceutical composition comprises at least one additional peptide which binds to class I molecules of the major histocompatibility complex (MHC class I molecules) and/or induces T cells cross-reacting with said peptide, wherein said at least one additional peptide comprises an amino acid sequence which is selected from the group consisting of: SEQ ID NO: 15 to SEQ ID NO: 62 and variant sequences thereof which are at least 88% homologous to SEQ ID NO: 15 to SEQ ID NO: 62, wherein said peptide is not a full-length polypeptide.

This measure has the advantage that the pharmaceutical composition of the invention is supplemented with MHC/HLA class I peptides resulting in a (semi-) individualized or personalized composition where the additional peptide(s) is selected in accordance with the MCH/HLA allotype of the patient.

In another embodiment of the invention the at least one additional peptide is selected depending on the MHC class I allotype of the individual to be treated, preferably the at least one additional peptide is selected according to the following table 4, wherein the amino acid sequences of each MHC class I allotype group includes variant sequences which are at least 88% homologous:

**Table 4: Selection of additional peptide(s) depending on the MHC class I allotype of the individual to be treated.**

| MHC class I allotype | Amino acid sequence of peptide (at least one of each allotype group) |
|---|---|
| A*02:01 | SEQ ID NOS: 15, 16, 17, 18, 19, 20, 21, 22, 23, 24 |
| A*01:01 | SEQ ID NOS: 25, 26, 27, 28, 29, 30, 31 |
| A*03:01 | SEQ ID NOS: 32 |
| A*24:02 | SEQ ID NOS: 33, 34, 35 |
| A*11:01 | SEQ ID NOS: 36 |
| B*07:02 | SEQ ID NOS: 37, 38 |
| B*51:01 | SEQ ID NOS: 39, 40, 41 |
| B*08:01 | SEQ ID NOS: 42, 43, 44, 45 |
| B*44:02 | SEQ ID NOS: 46, 47, 48 |
| B*40:01 | SEQ ID NOS: 49, 50, 51, 52, 53 |
| C*07:01 | SEQ ID NOS: 54, 55 |
| C*07:02 | SEQ ID NOS: 56 |
| C*04:01 | SEQ ID NOS: 57, 58 |
| C*03:04 | SEQ ID NOS: 59, 60, 61 |
| A*02:01 | SEQ ID NOS: 62 |

By this measure, there is provided a pharmaceutical composition comprising, in addition to one or more of the MHC class II peptides according to the invention (SEQ ID NOS: 1-14), one or more further peptides specifically targeting the MHC allotype of the individual to be treated. This provides a (semi-) personalized pharmaceutical composition that elicits an optimal and maximized immune response in the individual. The pharmaceutical composition is thereby particularly effective in or against a disease of OPSCC.

Against this background, in an embodiment the peptide according to the invention has the ability to bind to an MHC class I or II molecule, and wherein said peptide, when bound to said MHC, is capable of being recognized by CD4 and/or CD8 T cells. Preferably the amino acid sequence thereof comprises a continuous stretch of amino acids according to any one of SEQ ID NO: 1 to SEQ ID NO: 62.

In yet a further embodiment of the pharmaceutical composition according to the invention wherein it comprises at least 2, preferably at least 3, further preferably at least 4, further preferably at least 5, and highly preferably at least 6 different additional peptides, each additional peptide comprising an amino acid sequence which is selected from the group consisting of: SEQ ID NO: 15 to SEQ ID NO: 62 and variant sequences thereof which are at least 88% homologous to SEQ ID NO: 15 to SEQ ID NO: 62, wherein said additional peptide is not a full-length polypeptide.

As the number of additional peptides increases, the immunostimulatory activity of the pharmaceutical composition of the invention is increasingly enhanced in an allotype-specific manner.

In a still further embodiment of the invention said pharmaceutical composition is a vaccine.

Another subject-matter of the invention is an antibody, in particular a soluble or membrane-bound antibody, preferably a monoclonal antibody or fragment thereof, that specifically recognizes the peptide(s) or variant thereof according to the invention, preferably the peptide or variant thereof according to any of claims 9 to 11 when bound to an MHC molecule.

The term "antibody" or "antibodies" is used herein in a broad sense and includes both polyclonal and monoclonal antibodies. In addition to intact or "full" immunoglobulin molecules, also included in the term "antibodies" are fragments (e.g. CDRs, Fv, Fab and Fc fragments) or polymers of those immunoglobulin molecules and humanized versions of immunoglobulin molecules, as long as they exhibit any of the desired properties, i.e. specifically recognize the peptide(s) or variant thereof according to the invention. Whenever possible, the antibodies of the invention may be purchased from commercial sources. The antibodies of the invention may also be generated using well-known methods.

The features, characteristics, advantages and embodiments disclosed for the pharmaceutical composition and/or the peptide(s) according to the invention apply to the antibody and fragment thereof correspondingly.

Another subject-matter of the invention relates to a T cell receptor, preferably soluble or membrane-bound, or a fragment thereof, that is reactive with an HLA ligand, wherein said ligand is the peptide(s) or variant thereof according to the invention, preferably when bound to an MHC molecule.

The term "T cell receptor" (abbreviated TCR) according to the invention refers to a heterodimeric molecule comprising an alpha polypeptide chain (alpha chain) and a beta polypeptide chain (beta chain), wherein the heterodimeric receptor is capable of binding to a peptide antigen presented by an HLA molecule. The term also includes so-called gamma/delta TCRs.

The features, characteristics, advantages and embodiments disclosed for the peptide and antibody or fragment thereof according to the invention apply to the T cell receptor correspondingly.

A still further subject-matter according to the invention relates to a nucleic acid, encoding for a peptide or variant thereof according to the invention, an antibody or fragment thereof according to the invention, a T cell receptor or fragment thereof according to the invention, optionally linked to a heterologous promoter sequence, or an expression vector expressing said nucleic acid.

The nucleic acid coding for a particular peptide, oligopeptide, or polypeptide may be naturally occurring or it may be synthetically constructed. The nucleic acid (for example a polynucleotide) may be, for example, DNA, cDNA, PNA, RNA or combinations thereof, either single- and/or double- stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing a/the (poly)peptide(s) according to the invention.

As used herein the term "nucleic acid coding for (or encoding) a peptide" refers to a nucleotide sequence coding for the peptide including artificial (man-made) start and stop codons compatible for the biological system the sequence is to be expressed by, for example, a dendritic cell or another cell system useful for the production of TCRs. The term "promoter" means a region of DNA involved in binding of RNA polymerase to initiate transcription.

In an embodiment of the invention the nucleic acid is isolated. The term "isolated" means that the material is removed from its original environment (e.g., the natural environment, if it is naturally occurring). For example, a naturally-occurring polynucleotide or (poly-) peptide present in a living animal is not isolated, but the same polynucleotide or polypeptide, separated from some or all of the coexisting materials in the natural system, is isolated. Such polynucleotides could be part of a vector and/or such polynucleotides or (poly-) peptides could be part of a composition, and still be isolated in that such vector or composition is not part of its natural environment.

The features, characteristics, advantages and embodiments disclosed for the pharmaceutical composition and/or the peptide(s) according to the invention apply to the nucleic acid and expression vector correspondingly.

Another subject-matter of the invention relates to a recombinant host cell comprising the peptide(s) according to the invention, the antibody or fragment thereof according to the invention, the T cell receptor or fragment thereof according to the invention or the nucleic acid or the expression vector according to the invention, wherein said host cell preferably is selected from a mammalian or human cell, further preferably from an antigen presenting cell, such as a dendritic cell, a T cell or an NK cell.

The features, characteristics, advantages and embodiments disclosed for the pharmaceutical composition and/or peptide(s) according to the invention apply to the host cell correspondingly.

A still further subject-matter of the invention relates to an *in vitro* method for producing activated T lymphocytes, the method comprising contacting in vitro T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to the invention.

The activated T cells that are directed against the peptide(s) of the invention are useful in therapy. Thus, a further aspect of the invention provides activated T cells obtainable by the foregoing methods of the invention.

Activated T cells, which are produced by the above method, will selectively recognize a cell that aberrantly expresses a polypeptide that comprises an amino acid sequence of SEQ ID NO: 1 to SEQ ID NO: 62.

The features, characteristics, advantages and embodiments disclosed for the pharmaceutical composition and/or the peptide(s) according to the invention apply to the above method correspondingly.

Another subject-matter according to the invention relates to a pharmaceutical composition comprising at least one active ingredient selected from the group consisting of the peptide according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention, or a conjugated or labelled active ingredient, and a pharmaceutically acceptable carrier, and optionally, pharmaceutically acceptable excipients and/or stabilizers.

The features, characteristics, advantages and embodiments disclosed for the pharmaceutical composition recited at the outset and/or the peptide(s) according to the invention apply to said pharmaceutical composition correspondingly.

Another subject-matter of the invention relates to the peptide(s) according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention for use in medicine, preferably in the diagnosis and/or prevention and/or treatment of cancer, or for use in the manufacture of a medicament against cancer, preferably a vaccine, further preferably said cancer is oropharyngeal squamous cell carcinoma (OPSCC) and other tumors that show an overexpression of a protein from which a peptide SEQ ID NO: 1 to SEQ ID NO: 62 is derived from.

The features, characteristics, advantages and embodiments disclosed for the pharmaceutical composition and/or the peptide(s) according to the invention apply to the above-referenced use correspondingly.

A still further subject-matter according to the invention relates to a kit comprising:
a) a container comprising a pharmaceutical composition according to the invention, the peptide(s) or the variant according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention, in solution or in lyophilized formulation;
b) optionally, a second container containing a diluent or reconstituting solution for the lyophilized formulation;
c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kits of the present invention preferably comprises a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments disclosed for the pharmaceutical composition and/or the peptide(s) according to the invention apply to the kit correspondingly.

Another subject-matter of the invention relates to a method for producing a personalized anti-cancer pharmaceutical composition, preferably a vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or overpresentation in tumors as compared to normal tissues;
c) selecting at least one peptide from the warehouse that matched a TUMAP identified in said patient; and
d) formulating the personalized vaccine based on step c),
wherein said warehouse comprises a plurality of peptides and/or variant sequences according to the invention.

A "personalized pharmaceutical composition" shall mean specifically tailored therapies for one individual patient that will only be used for therapy in such individual patient, including actively personalized cancer vaccines and adoptive cellular therapies using autologous patient tissue.

As used herein, the term "warehouse" shall refer to the peptides according to the invention that have been pre-screened for immunogenicity and/or over-presentation in a particular tumor type, in particular oropharyngeal squamous cell carcinoma (OPSCC). The warehouse (e.g. in the form of a data-base) is composed of tumor-associated peptides which were highly overexpressed in cancer cells of patients with various HLA-A, HLA-B and HLA-C alleles. It contains MHC class I and MHC class II peptides. In particular, it contains MHC class I A*02:01, A*01:01, A*03:01, A*24:02, A*11:01, B*07:02, B*51:01, B*08:01, B*44:02, B*40:01, C*07:01, C*07:02, C*04:01, C*03:04 und C*06:02 marker peptides. These peptides allow comparison of the magnitude of T-cell immunity induced by TUMAPS in a quantitative manner and hence allow important conclusion to be drawn on the capacity of the vaccine to elicit anti-tumor responses.

In one exemplary embodiment, the peptides included in the vaccine are identified by: (a) identifying tumor-associated peptides (TUMAPs) presented by a tumor sample from the individual patient by the method as described above; (b) comparing the peptides identified in a) with the warehouse of peptides that have been prescreened for immunogenicity and overpresentation in tumors as compared to corresponding normal tissue; (c) selecting at least one peptide from the warehouse that correlates with a tumor-associated peptide identified in the patient; and (d) optionally, selecting at least one peptide identified *de novo* in (a) confirming its immunogenicity.

Once the peptides for a personalized peptide based pharmaceutical composition, e.g. a vaccine, are selected, the composition is produced. The composition or vaccine preferably is a liquid formulation consisting of the individual peptides dissolved in between 20-40% DMSO, preferably about 30-35% DMSO, such as about 33% DMSO.

The features, characteristics, advantages and embodiments disclosed for the pharmaceutical composition and/or the peptide(s) according to the invention apply to the afore-referenced method correspondingly.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:

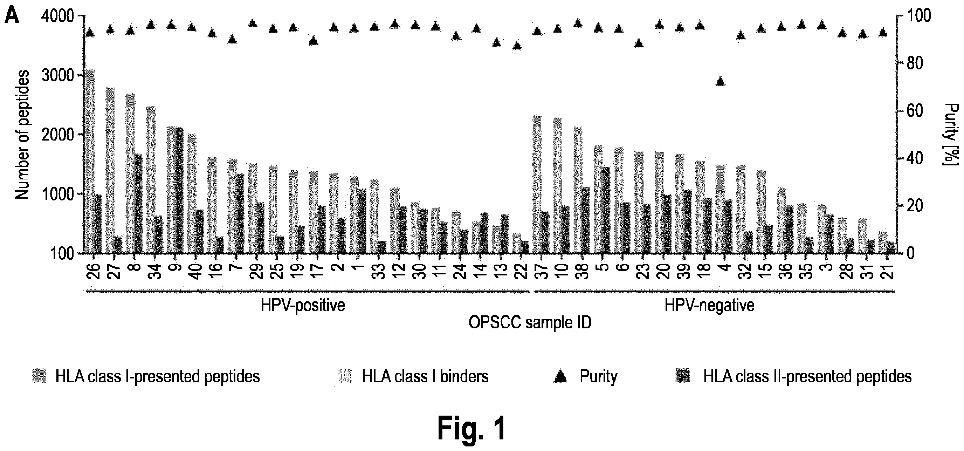

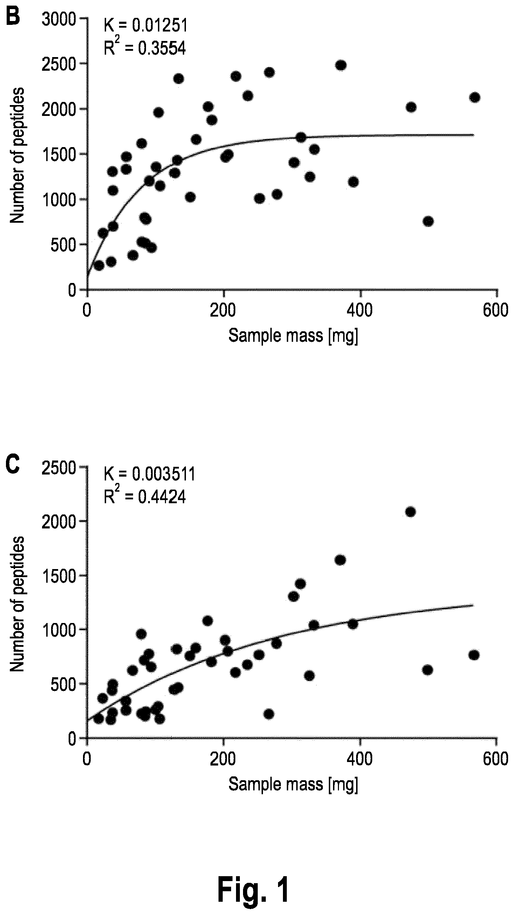

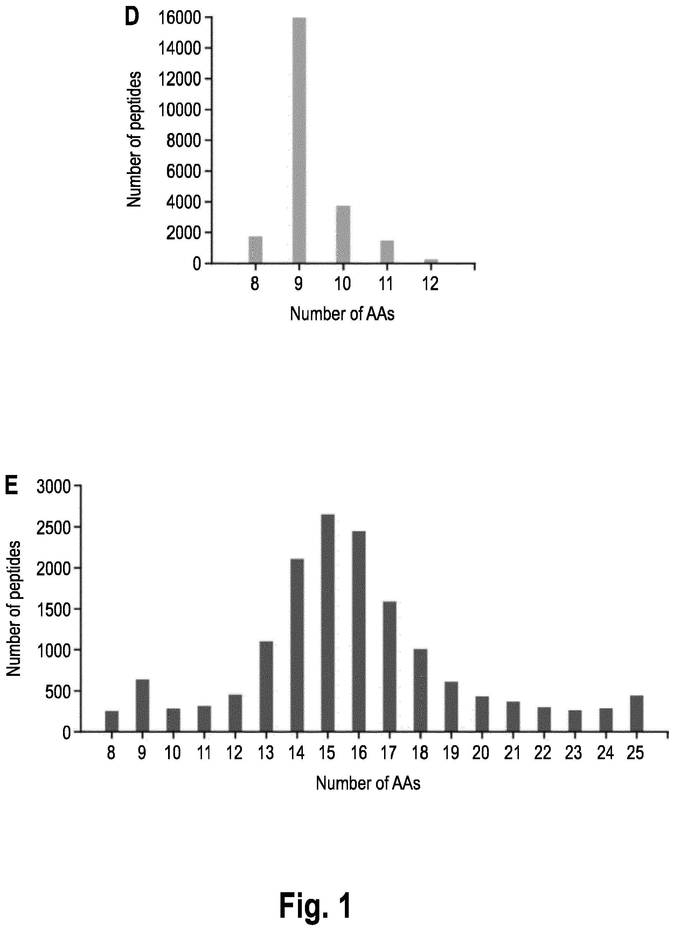

- Fig. 1:: (A) HLA class I and (B) HLA class II allotype population coverage within the OPSCC patient cohort compared to the world population (calculated by the IEDB population coverage tool, www.iedb.org) [1]. The frequencies of individuals within the world population carrying up to six HLA allotypes (x-axis) of the respective CML or benign dataset are indicated as grey bars on the left y-axis. The cumulative percentage of population coverage is depicted as black dots on the right y-axis. (C) Saturation analysis of HLA class I ligand source proteins and (D) the source proteins of HLA class II-presented peptides of the OPSCC patient cohort. Number of unique source protein identifications are shown as a function of cumulative HLA ligandome analysis of OPSCC samples (n = 40). Exponential regression allowed for the robust calculation (R2 = 0.9933 and R = 0.9985) of the maximum attainable number of different source protein identifications (black line). The dashed line depicts the source proteome coverage achieved in the OPSCC patient cohort. (E) HLA class I and (F) HLA class II peptide presentation were calculated for different presentation frequencies. The process of peptide randomization, cohort assembly and tumor-associated peptide identification was repeated 1,000 times and the mean value of resulting decoy identifications was calculated and plotted for the different threshold values together with the real peptide identifications. The corresponding FDRs for any chosen tumor-associated peptide threshold are listed below the x-axis. OPSCC - oropharyngeal squamous cell carcinoma; n(OPSCCs) = 40; FDR - false discovery rate.

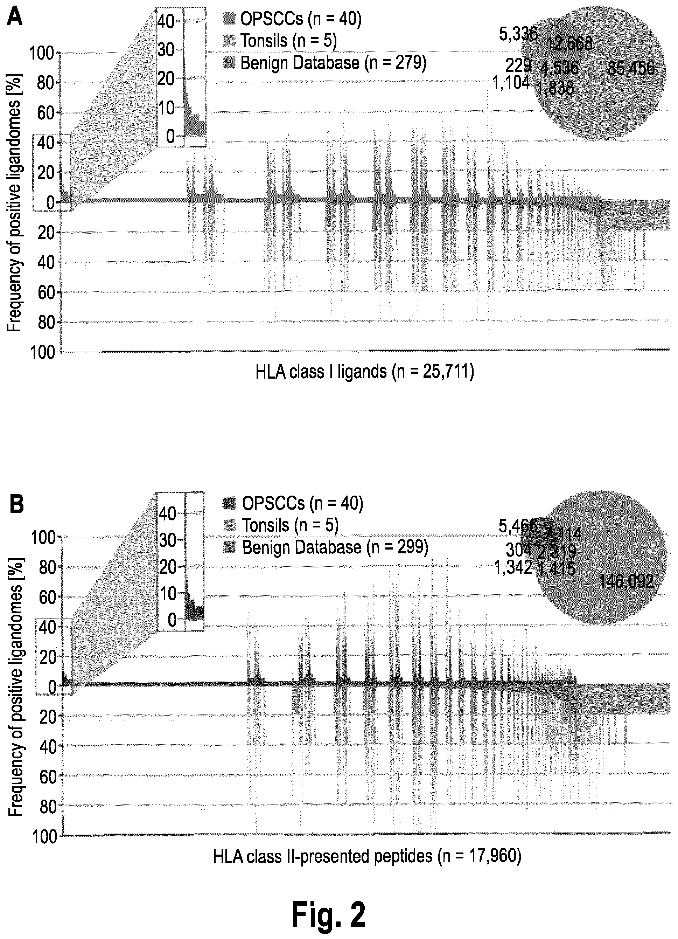

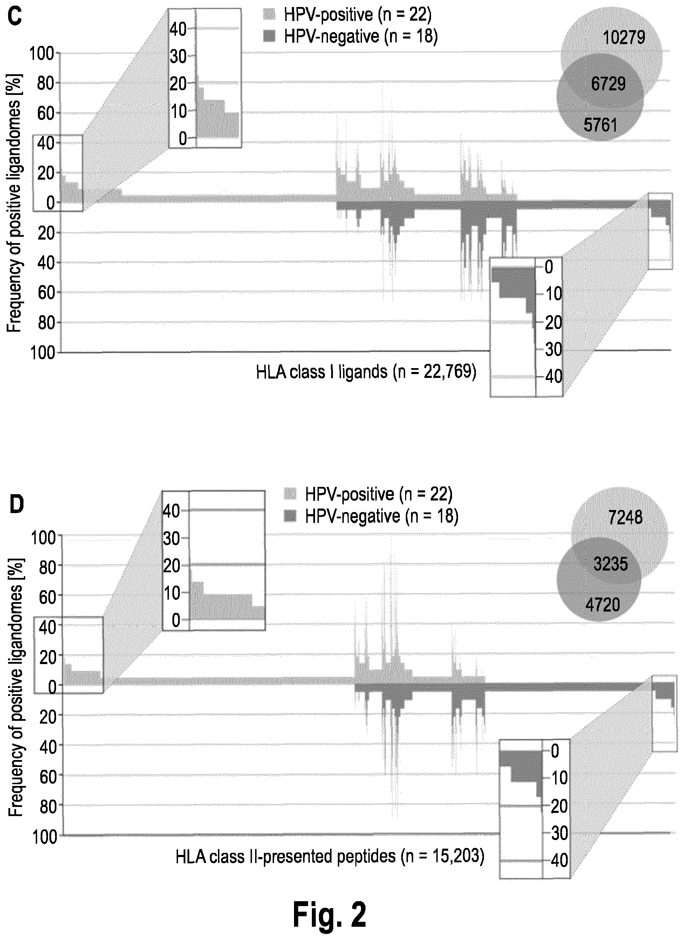

- Fig. 2: (A) HLA-A, (B) HLA-B, (C) HLA-C and (D) HLA-DRB1 allelic distribution in the OPSCC patient cohort and a German reference population ("Germany pop 8", Allele Frequency Net Database AFND, www.allelefrequencies.net). Top 5 assigns the five most frequent alleles within the patient cohort. OPSCC - oropharyngeal squamous cell carcinoma; n(Patients) = 40; n(Germany) = 39,689; * - p-value ≤ 0.05 after Bonferroni correction.

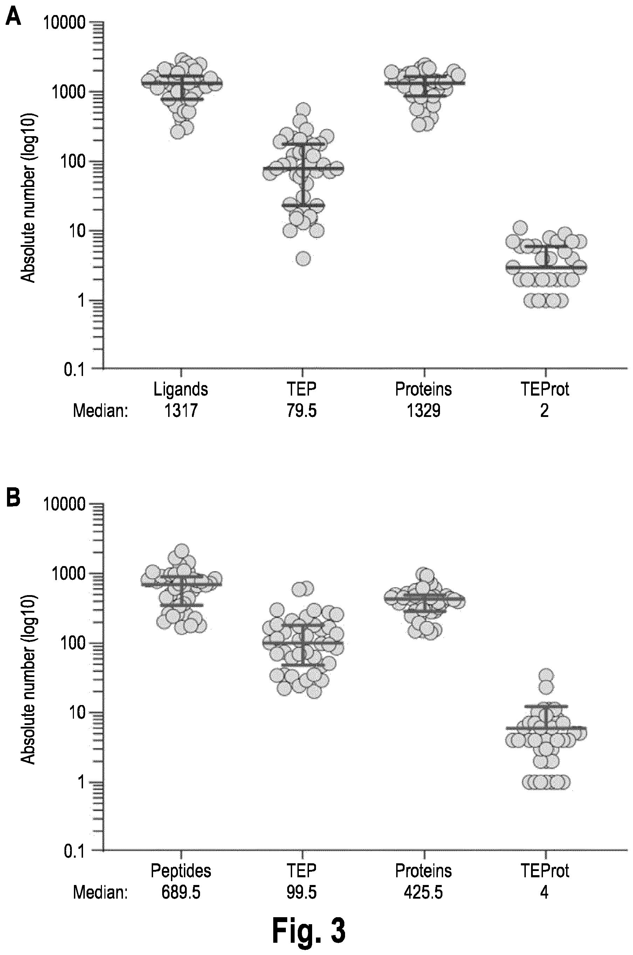

- Fig. 3: (A) Yields of isolated HLA class I- and class II-presented peptides for individual OPSCC samples achieved by LC-MS/MS analysis. Peptide yields varied between 265 and 2,854 HLA class I- (mean = 1,424) and between 168 and 2,086 HLA class II-presented peptides (mean = 702) per individual sample. HLA class I ligands were defined as HLA class I-presented peptides carrying a binding motif of an HLA allotype of the respective patient using SYFPEITHI and NetMHCpan 4.0. The purity is defined as the proportion of binders among all HLA class I-presented peptides and indicated by black triangles on the right y-axis. Correlation of sample masses and yields of (B) HLA class I ligands and (C) HLA class II-presented peptides. Length distribution analysis of (D) HLA class I ligands and (E) HLA class II-presented peptides. OPSCC - oropharyngeal squamous cell carcinoma; n(OPSCCs) = 40; AA - amino acid.

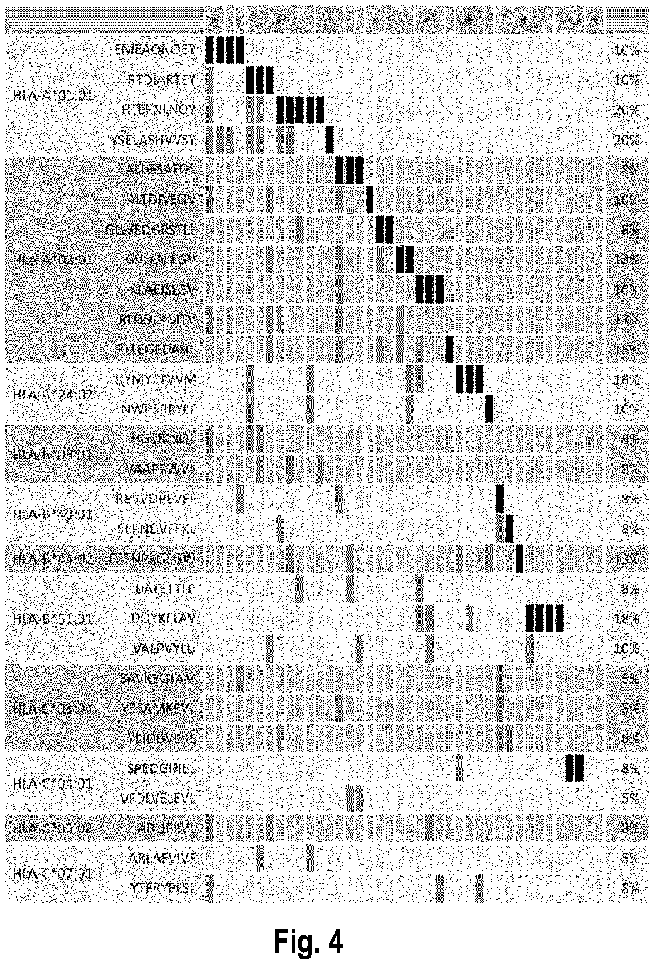

- Fig. 4: Comparative profiling of (A) HLA class I ligands and (B) HLA class II-presented peptides based on the frequency of presentation in OPSCC and benign ligandomes. Comparative profiling of (C) HLA class I ligands and (D) HLA class II-presented peptides based on the frequency of presentation in HPV-positive and HPV-negative OPSCC ligandomes. Frequencies of positive immunopeptidomes for the respective peptides (x-axis) are indicated on the y-axis.

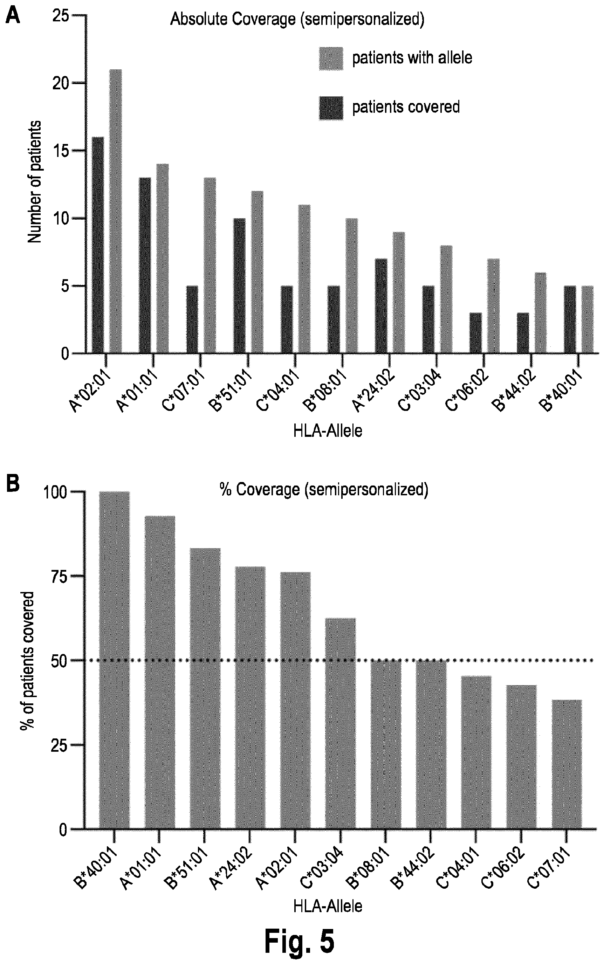

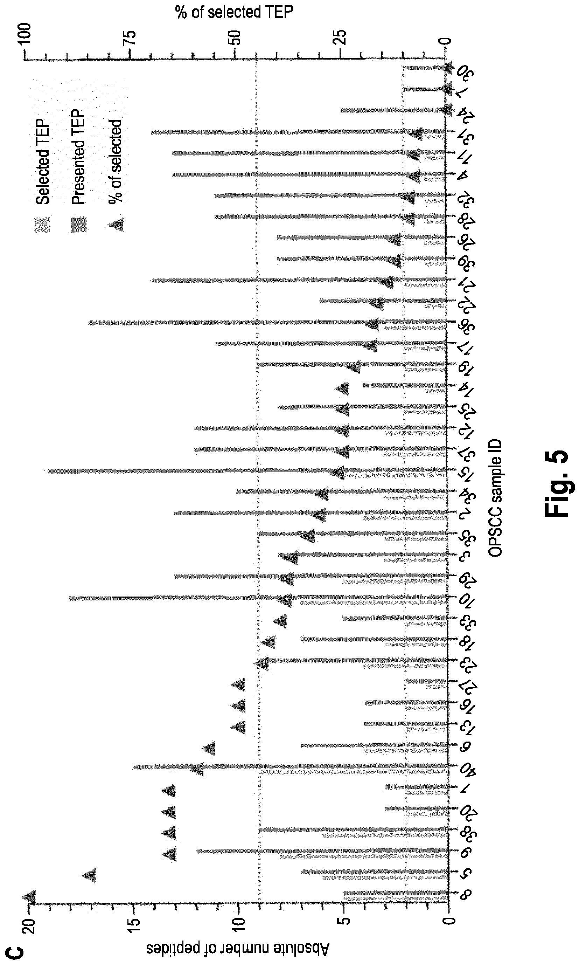

- Fig. 5: Scatter plot of the supervised PCA on the basis of HLA ligands identified in HPV⁺ and HPV⁻ OPSCCs. The PCA was based on the merged source proteins of HLA class I binders and HLA class II-presented peptides from OPSCC samples. n(HPV-positive samples) = 22; n(HPV-negative samples) = 18; n(high-impact proteins) = 190; PCA = principle component analysis.

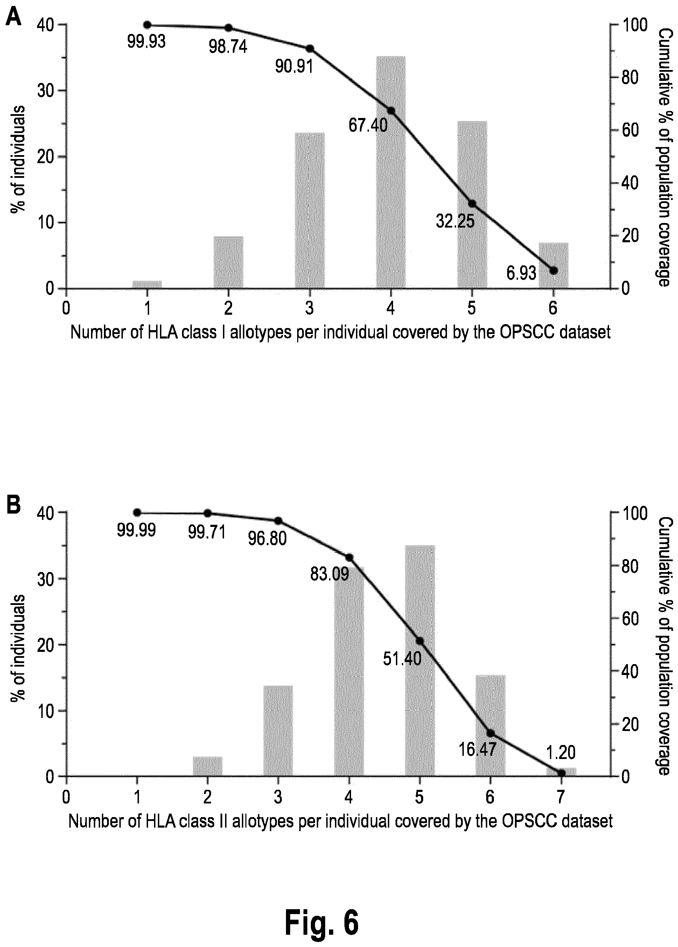

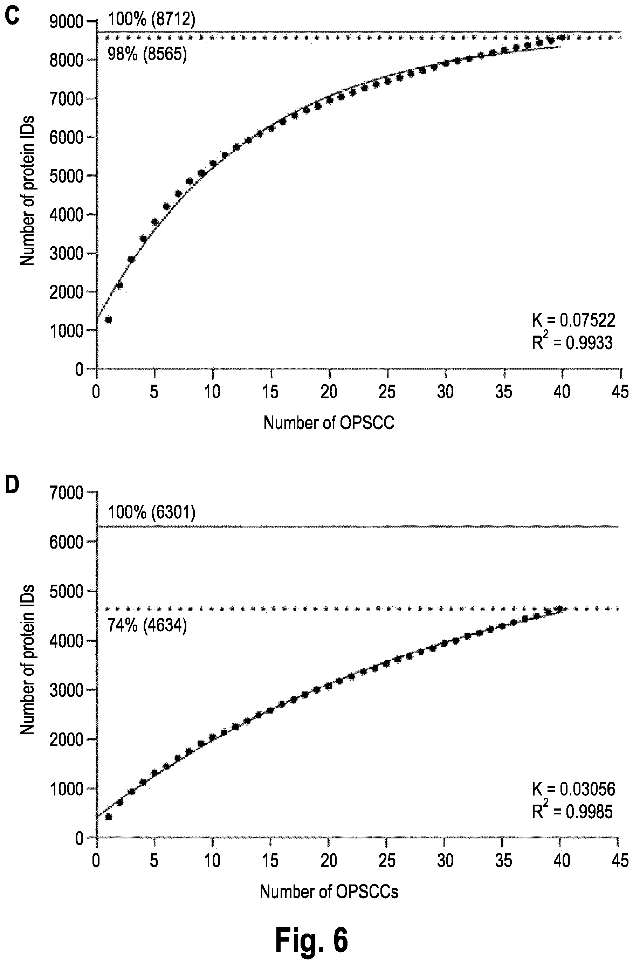

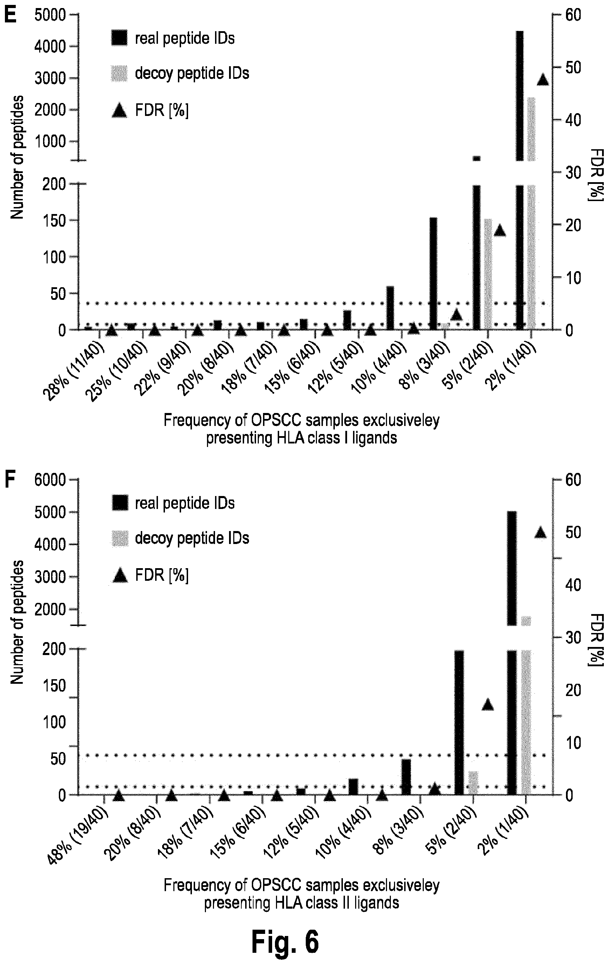

- Fig. 6: Absolute numbers of HLA ligands or -presented peptides, tumor-exclusive (TEP) peptides, source proteins and tumor-exclusive proteins (TEProt) for (A) HLA class I and (B) HLA class II on a logarithmic scale (log10). Red lines represent the median and interquartile range. The median values are indicated below the x-axis labels.

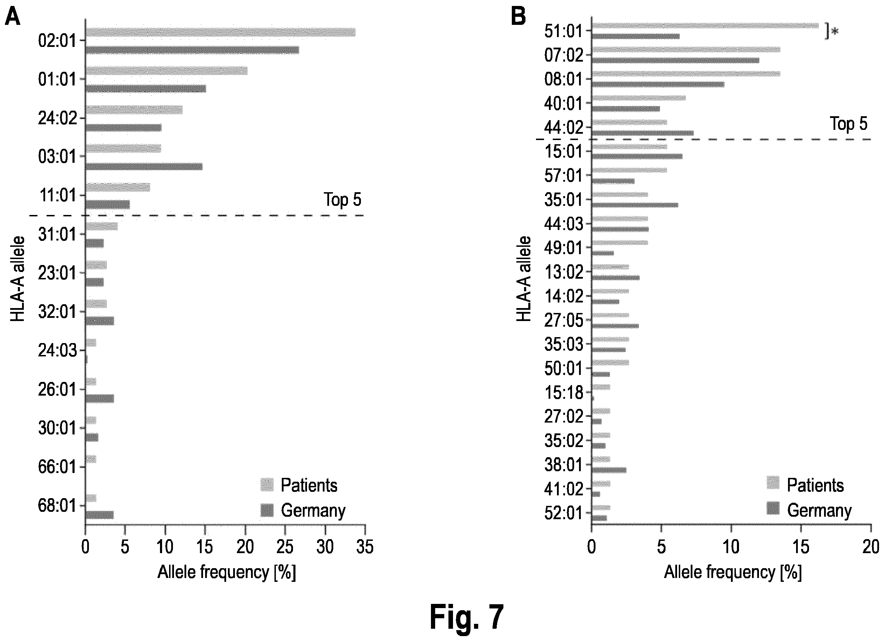

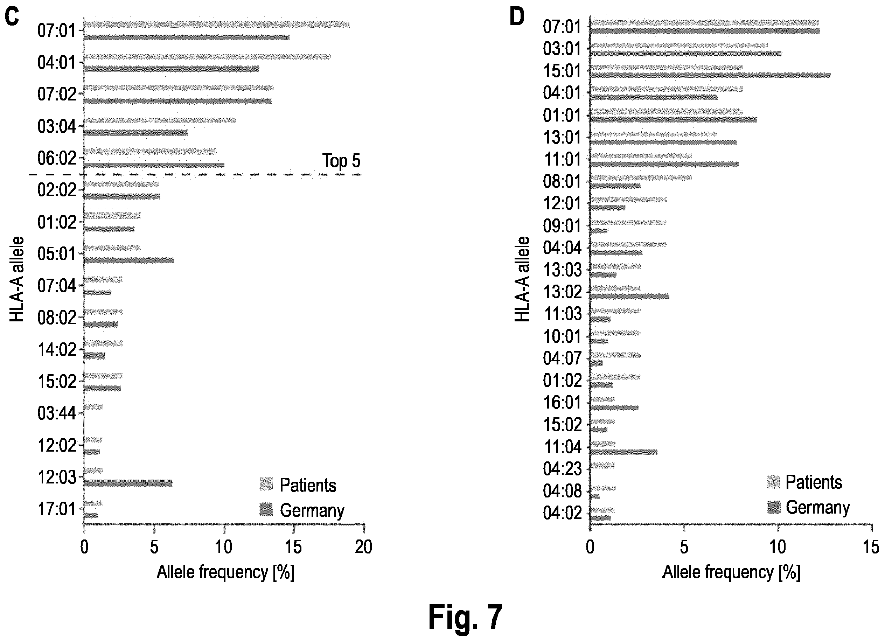

- Fig. 7: Warehouse of TEP for 11 HLA allotypes. The HLA allotype is indicated in the left column, the next column shows the peptide amino acid sequence. Patients are presented in columns and peptide coverage is indicated by dark bars. Black bars indicate samples that contribute directly to coverage, whereas overlap is indicated by grey bars. Patient HPV status is indicated in the top line (red - HPV-positive (+), blue - HPV-negative (-)). The right column indicates peptide prevalence in relation to the whole cohort (n = 40). The graph was generated using AVAtar

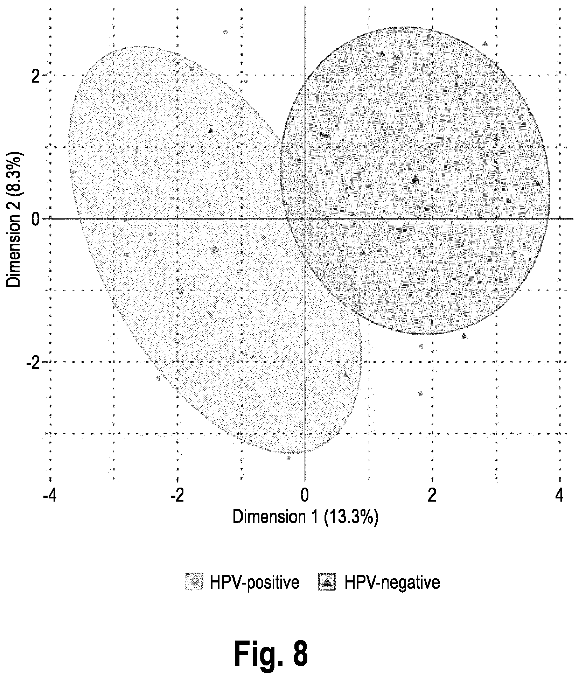

- Fig. 8: Coverage and overlap of the HLA-specific peptide selections by HPV-status. The peptide selections for the respective HLA-alleles were merged and are displayed in rows and individual patients in columns. The coverage for the respective peptide selection is indicated in the last column. Using all 29 peptides, all HPV-negative patients and 90.9% of HPV-positive patients were covered. Overlap was 1.167 for HPV-negative patients and 1.0 for HPV-positive patients.

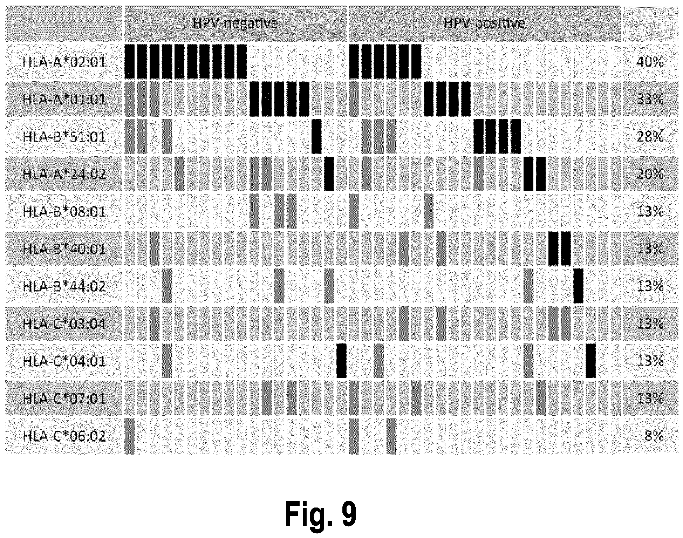

- Fig. 9: Patient coverage by semi-personalized warehouse peptide selections. (A) Bar graph indicating the absolute number of patients with the respective HLA allele (grey) and patients presenting at least one of the semi-personalized peptides selected (red) ordered by the prevalence of the respective allele in the cohort. (B) Bar graph showing the relative number of patients covered by at least one peptide of the semi-personalized peptide selection ordered from highest coverage to lowest. (C) Bar graph indicating the absolute number of peptides selected (grey bars) for the semipersonalized TEP selection for each individual patient based on its HLA typing and the absolute number of actually presented peptides selected (red) graphed on the left y-axis. The right y-axis displays the relative number of presented peptides in relation to the number of peptides selected (% of selected TEP, triangles). Patients were ordered by descending coverage.

### 1. Material and Methods

### Patients

Fresh frozen tissue samples derived from 22 HPV-positive and 18 HPV-negative patients were prospectively collected at Ulm University Medical Center before treatment initiation. HPV status was determined via RNA-Seq (compare below).

Among the 22 HPV-positive patients, 19 were associated with HPV-16 and the other three with HPV-35, HPV-58 or HPV-59, respectively. OPSCC tumor biopsies and tonsillar tissue samples from 5 five healthy donors were surgically resected, immediately snap-frozen in liquid nitrogen (N2) and subsequently stored at -80 °C. Written informed consent was obtained in accordance with the Declaration of Helsinki protocol. The study was performed according to the guidelines of the local ethics committee (222/13, 90/15). Patient characteristics are provided in table 5.

**Table 5: Patient characteristics. HPV = Human Papillomavirus; ENE = extranodal extension; BOT = base of tongue**

| | | HPV-negative | % | HPV-positive | % | Total | % |
|---|---|---|---|---|---|---|---|
| Total | | 18 | 45.0% | 22 | 55.0% | 40 | 100.0% |
| Sex | male | 12 | 30.0% | 21 | 52.5% | 33 | 82.5% |
| | female | 6 | 15.0% | 1 | 2.5% | 7 | 17.5% |
| age | | 59,6 | n.a. | 60,2 | n.a. | 59.9 | n.a. |
| pT | pT1 / T2 | 7 | 17.5% | 11 | 27.5% | 18 | 45.0% |
| | pT3 / T4 | 10 | 25.0% | 11 | 27.5% | 21 | 52.5% |
| | missing | 1 | 2.5% | 0 | 0.0% | 1 | 2.5% |
| pN | pN0 | 6 | 15.0% | 8 | 20.0% | 14 | 35.0% |
| | pN1 | 4 | 10.0% | 6 | 15.0% | 10 | 25.0% |
| | pN2 | 2 | 5.0% | 7 | 17.5% | 9 | 22.5% |
| | pN3a/b | 5 | 12.5% | 1 | 2.5% | 6 | 15.0% |
| | missing | 1 | 2.5% | 0 | 0.0% | 1 | 2.5% |
| Stage | I | 1 | 2.5% | 7 | 17.5% | 8 | 20.0% |
| (UICC v8) | II | 3 | 7.5% | 10 | 25.0% | 13 | 32.5% |
| | III | 6 | 15.0% | 3 | 7.5% | 9 | 22.5% |
| | IV (a/b) | 8 | 20.0% | 2 | 5.0% | 10 | 25.0% |
| R-status | R0 | 13 | 32.5% | 20 | 50.0% | 33 | 82.5% |
| | R1 | 1 | 2.5% | 1 | 2.5% | 2 | 5.0% |
| | close margin (<5mm) | 3 | 7.5% | 1 | 2.5% | 4 | 10.0% |
| L-status | L0 | 9 | 22.5% | 12 | 30.0% | 21 | 52.5% |
| | L1 | 8 | 20.0% | 10 | 25.0% | 18 | 45.0% |
| | missing | 1 | 2.5% | 0 | 0.0% | 1 | 2.5% |
| V-status | V0 | 17 | 42.5% | 20 | 50.0% | 37 | 92.5% |
| | V1 | 0 | 0.0% | 2 | 5.0% | 2 | 5.0% |
| | missing | 1 | 2.5% | 0 | 0.0% | 1 | 2.5% |
| Pn-status | Pn0 | 11 | 27.5% | 20 | 50.0% | 31 | 77.5% |
| | Pn1 | 6 | 15.0% | 2 | 5.0% | 8 | 20.0% |
| | missing | 1 | 2.5% | 0 | 0.0% | 1 | 2.5% |
| ENE | ENE-negative | 5 | 12.5% | 3 | 7.5% | 8 | 20.0% |
| | ENE-positive | 6 | 15.0% | 10 | 25.0% | 16 | 40.0% |
| Grading | well differentiated | 1 | 2.5% | 0 | 0.0% | 1 | 2.5% |
| | moderately differentiated | 8 | 20.0% | 6 | 15.0% | 14 | 35.0% |
| | poorly differentiated | 9 | 22.5% | 16 | 40.0% | 25 | 62.5% |
| | undifferentiated | 0 | 0.0% | 0 | 0.0% | 0 | 0.0% |
| Subsite | BOT | 4 | 10.0% | 1 | 2.5% | 5 | 12.5% |
| | soft palate | 1 | 2.5% | 0 | 0.0% | 1 | 2.5% |
| | tonsil | 13 | 32.5% | 21 | 52.5% | 34 | 85.0% |
| smoking history | never smoker | 1 | 2.5% | 3 | 7.5% | 4 | 10.0% |
| | former smoker | 6 | 15.0% | 12 | 30.0% | 18 | 45.0% |
| | current smoker | 11 | 27.5% | 6 | 15.0% | 17 | 42.5% |
| | missing | 0 | 0.0% | 0 | 0.0% | 0 | 0.0% |
| | pack years (mean) | 30 | n.a. | 20 | n.a. | 24.8 | n.a. |
| alcohol consumption | never | 0 | 0.0% | 1 | 2.5% | 1 | 2.5% |
| | sometimes | 5 | 12.5% | 14 | 35.0% | 19 | 47.5% |
| | daily | 8 | 20.0% | 4 | 10.0% | 12 | 30.0% |
| | intensively_daily | 2 | 5.0% | 0 | 0.0% | 2 | 5.0% |
| | former heavy drinker | 3 | 7.5% | 2 | 5.0% | 5 | 12.5% |
| | former consumption | 0 | 0.0% | 0 | 0.0% | 0 | 0.0% |
| | # drinks per week (mean) | 32 | n.a. | 23 | n.a. | 30 | n.a. |

### HLA typing

High resolution HLA-typing for the loci HLA-A, -B, -C, -DRB1, -DQB1, and -DPB1 was performed using an in-house developed IVD-CE-certified NGS-amplicon sequencing protocol (H-Seq-LR-NGS, DRK-BSD, Baden-Württemberg Hessen, Ulm), based on the Illumina MiSeq platform (San Diego, CA, USA) or based on the HLA binding motifs of isolated HLA-presented peptides using SYFPEITHI (Rammensee et al. SYFPEITHI: database for MHC ligands and peptide motifs. Immunogenetics 50 (3-4), 213-219 (1999)) and NetMHC (Andreatta M and Nielsen M. Gapped sequence alignment using artificial neural networks: application to the MHC class I system. Bioinformatics 32 (4), 511-517 (2016)).

### Isolation of HLA-presented peptides

HLA class I- and class II-presented peptides were isolated from tissue samples performing standard immunoaffinity purification as previously described. The HLA class I A, B, and C-specific monoclonal antibody (mAb) W6/32, the pan-HLA class II-specific mAb Tü-39, and the HLA-DR-specific mAb L243 (all produced in-house) were used to extract HLA molecules.

### Analysis of HLA ligands by liquid chromatography-tandem MS

HLA ligand extracts were analyzed as previously described. HLA ligands and were separated using reversed-phase ultra-high performance liquid chromatography (nanoUHPLC, UltiMate 3000 RSLCnano, Dionex). Eluted peptides were analyzed by tandem mass spectrometry (MS/MS) in an on-line coupled LTQ Orbitrap XL hybrid mass spectrometer (Thermo Fisher Scientific) equipped with a nano-electron spray ion source.

### Data processing

Processing of MS data was performed using the Proteome Discoverer 1.4 software (Thermo Fisher Scientific). Database search and spectral annotation were performed against the human proteome as comprised in the UniProtKB/Swiss-Prot data-base (20,279 reviewed protein sequences; September 27th, 2013; www.uniprot.org) via the SequestHT algorithm. Search for HLA-presented peptides derived from viral proteins was based on the HPV proteome as comprised in the UniProtKB/Swiss-Prot database (470 reviewed protein sequences; 5 January 2018; www.uniprot.org). Mass tolerance for processing was set to 5 ppm for precursor ions and 0.5 Da for fragment ions. Oxidized methionine was allowed as only dynamic modification and no cleavage specificity was selected. Peptide identifications were filtered using the Percolator 2.04 with a target value of q ≤ 0.05 (5% FDR). Additional filters for search engine rank (= 1) and peptide length (= 8-25 amino acids) were applied. HLA class I ligand annotation was performed using SYFPEITHI and NetMHCpan 4.0 (Andreatta M and Nielsen M, *l.c.*)*.*

### HPV typing/RNA sequencing

For this analysis, RNA sequencing data were only used to define HPV-status. Total RNA was extracted using AIIPrep DNA / RNA Mini Kit (Qiagen, Germany) from fresh, snap-frozen tumor samples. Sequencing of RNA samples was performed using Illumina's next-generation sequencing methodology. In detail, total RNA was quantified and quality checked using Agilent 2100 Bioanalyzer Instrument (Agilent RNA 6000 Pico). Libraries were prepared from 500 ng of input material using TruSeq Stranded mRNA (manufacturer's instructions) and subsequently quantified and quality checked using Agilent 2100 Bioanalyzer Instrument (DNA 7500 kit). Libraries were pooled and sequenced in one lane of HiSeq 2500 System running in 51 cycle/single-end/high output mode. Sequence information was converted to FASTQ format using bcl2fastq (2.20.0.422). High-quality SE reads were mapped to the human genome (hg38) using STAR (2.0.9) and, following the removal of multimapping reads, converted to gene specific read counts for annotated genes using featureCounts (2.0.0). Unmapped reads to the human genome were aligned to HPV high-risk type genomes using a viGen bioinformatic pipeline. Samples with ≥ 500 reads for HPV E6 or E7 RNA or ≥ 500 reads for all HPV oncogenes (E1, E2, E4, E5, E6, E7, L1, L2) in summary were considered HPV-positive. The HPV type with the highest number of reads was selected. HPV-negative cases had a mean of 8 reads for E1, E2, E4, E5, E6, E7, L1, L2 in summary (range: 0-23). (HPV RNA reads for HPV-16, -18, -35, -58 and -59: data not shown). In addition to RNA sequencing, data for p16 immunohistochemistry and HPV DNA PCR were available for all samples.

### Whole exome sequencing

DNA was extracted using the Qiagen AIIPrep DNA/RNA Mini Kit. Sequencing of exome samples was performed using Illumina's next-generation sequencing methodology. In detail, total DNA was quality checked using Agilent 4200 TapeStation System (Agilent Genomic DNA ScreenTape) and quantified using Quant-iT^{™} PicoGreen^{™}. Libraries were prepared from 3 µg of input material using SureSelect Human All Exon V6 (manufacturer's instructions) and subsequently quantified and quality checked using Agilent 4200 TapeStation System (D1000 ScreenTape). Libraries were pooled and sequenced on NextSeq 500 System (High Output Flow Cell) running in 150 cycle (2x 75bp paired-end) mode. Sequence information was converted to FASTQ format using bcl2fastq v2.20.0.422. The WES data were used to search for HLA-presented neoepitopes in the OPSCC HLA ligandome. Database search and spectral annotation were performed against the combination of the human proteome as comprised in the UniProtKB/Swiss-Prot database and the mutated protein sequences as defined for the respective patients.

### Software, statistical analysis and online tools

For overlapping analysis, the bioinformatics tools BioVenn and jVenn were used. The benign reference dataset used for comparative profiling was comprised of the HLA ligandome data of a previously reported hematological benign cohort (Bilich et al. The HLA ligandome landscape of chronic myeloid leukemia delineates novel T-cell epitopes for immunotherapy. Blood 133 (6), 550-565 (2019)), the benign tissue dataset provided within the HLA Ligand Atlas (Marcu et al. The HLA Ligand Atlas - A resource of natural HLA ligands presented on benign tissues (2019)) as well as additional in-house acquired HLA ligandome data of benign tissue and cell line samples. HLA ligandome data were also compared to previously published immunopeptidomes of other solid malignant diseases (ovarian cancer, hepatocellular carcinoma, renal cell carcinoma, glioblastoma). Statistical analysis was performed using the GraphPad Prism 6.1/9.01 software (GraphPad Software Inc).

The software AVAtar, previously developed at Ulm University was used to determine peptide combinations for the Top 5 HLA-HLA class I allotypes of HLA-A, -B and -C using multiobjective optimization of coverage for the respective HLA allotype and the number of antigens selected as previously described. Tumor-exclusive peptides (TEP) found in ≥ 2 patients of a certain class I allotype were filtered. These peptide candidates were subjected to multiobjective optimization with preset configurations and 1×10⁶ iterations for the HLA allotypes with the highest prevalence in the cohort. Each peptide that appeared in a selection underwent additional quality control (QC) to ensure sufficient goodness and specificity of fit between experimental and theoretical spectra. This entailed minimum requirements for the number of peptide spectrum matches (PSMs ≥ 2), the cross correlation value (Xcorr ≥ 1.5) and the delta correlation score between primary and the secondary sequence candidates (ΔCn ≥ 0.2). All peptides appearing up the optimization runs were considered potential candidates for a peptide warehouse. In total, up to 3 optimization runs were performed if peptides in the selection had to be removed after QC.

### 2. Results

The OPSCC patient study cohort (n = 40) comprised a total of 49 different HLA class I allotypes, covering at least one HLA class I allotype for 99.93% of individuals within the world's population (Figure 1A). Most frequent alleles within the patient cohort were HLA-A*02:01 (n = 25), HLA-A*01:01 (n = 15), HLA-C*07:01 (n = 14), HLA-C*04:01 (n = 13) and HLA-B*51:01 (n = 12). For each class I Isotype (HLA-A, -B, -C), the top 5 alleles were highlighted in Figure 1A and used for multiobjective optimization (compare below). The distribution of HLA class I allele frequencies was representative for most alleles in comparison to a German reference population (cohort "Germany pop 8" (n = 39,689); www.allelefrequencies.net). In contrast, the allele frequency of HLA-B*51:01 was significantly higher in OPSCC patients compared to the reference cohort, respectively (16.2% and 6.3%; p = 0.0005; OR = 2.9; 95% CI = 1.6 to 5.3) (Figure 2A-C).

LC-MS/MS-based analysis of the HLA class I ligandomes isolated from 40 OPSCC tissue samples identified a total of 22,769 unique HLA class I ligands (range: 265 - 2,854 peptides per sample; mean: 1,338 peptides per sample) derived from 9,485 different source proteins (Figure 3A, Table 6) and, thereby, obtaining 98% of the estimated maximum attainable coverage in HLA ligand source proteins (Figure 1C). A weak positive correlation between tissue sample masses and yields of HLA class I ligands was observed (p = 0.0019; Pearson's correlation coefficient r = 0.4645; 95% confidence interval (CI) = 0.2 to 0.7) (Figure 3B). As expected, the majority of HLA class I ligands (70%) exhibited a peptide length of 9 nine amino acids (Figure 3D).

33 different HLA-DRB, 13 different HLA-DQB and 12 different HLA-DPB allotypes were comprised in the OPSCC patient cohort, covering at least one1 HLA class II allotype in 99.99% of individuals among the world's population (Figure 1B). The most frequent alleles within the OPSCC cohort were HLA-DPB1*04:01 (n = 23), HLA-DQB1*03:01 (n = 17), HLA-DRB4*01:03 (n = 13) and HLA DPB1*03:01 (n = 12) in descending order. The comparison of the HLA-DRB1 allelic prevalence in the OPSCC patient cohort and a German reference population (cohort "Germany pop 8" (n = 39,689), www.allelefrequencies.net) revealed no significant differences in HLA class II allelic distribution (Figure 2D).

Mapping the HLA class II ligandomes of 40 OPSCC tissue samples by LC-MS/MS revealed a total of 15,203 unique HLA class II-presented peptides (range: 168 - 2,086 peptides per sample; mean: 702 peptides per sample) from 4,634 source proteins (Figure 3A; Table 6), achieving a 74% coverage of the estimated maximum attainable number of source proteins (Figure 1D). A positive correlation of sample masses and yields of HLA class II-presented peptides was shown (p < 0.0001; Pearson's correlation coefficient r = 0.6388; 95% CI = 0.4 to 0.8) (Figure 3C). Lengths of HLA class II-presented peptides were distributed across the tolerated range of 9 to 25 amino acids, with 15 amino acids as the most abundant peptide length (17%) (Figure 3E).

**Table 6: Overview of HLA ligandomic yields from OPSCCs. Isolated peptides were analyzed by LC-MS/MS. The HLA class I purity was calculated by the percentage of HLA class I ligands among the total number of HLA class I-presented peptides. The results of two biological replicates were merged for sample #26 and #27.**

| **Sam- ple num- ber** | **Tis- sue mass [mg]** | **HLA class I** | | | | | **HLA class II** | |
|---|---|---|---|---|---|---|---|---|
| | | **Pre- sented pep- tides** | **Source pro- teins** | **Bind- ers** | **Source pro- teins of bind- ers** | **Purity [%]** | **Pre- sented pep- tides** | **Source pro- teins** |
| **1** | 389 | 1253 | 1312 | 1192 | 1269 | 95 | 1050 | 600 |
| **2** | 326 | 1307 | 1362 | 1246 | 1310 | 95 | 574 | 391 |
| **3** | 132 | 1471 | 1481 | 1430 | 1446 | 97 | 820 | 473 |
| **4** | 371 | 2637 | 2311 | 2481 | 2204 | 94 | 1644 | 842 |
| **5** | 474 | 2092 | 1892 | 2019 | 1831 | 97 | 2086 | 920 |
| **6** | 303 | 1553 | 1502 | 1403 | 1386 | 90 | 1308 | 577 |
| **7** | 67 | 425 | 469 | 378 | 426 | 89 | 624 | 371 |
| **8** | 38 | 732 | 841 | 701 | 815 | 96 | 494 | 348 |
| **9** | 151 | 1059 | 1142 | 1025 | 1098 | 97 | 758 | 441 |
| **10** | 57 | 1578 | 1507 | 1468 | 1414 | 93 | 255 | 232 |
| **11** | 91 | 1336 | 1291 | 1200 | 1226 | 90 | 777 | 472 |
| **12** | 94 | 488 | 493 | 464 | 473 | 95 | 656 | 408 |
| **13** | 23 | 682 | 795 | 626 | 737 | 92 | 366 | 286 |
| **14** | 101 | 1431 | 1426 | 1355 | 1362 | 95 | 262 | 244 |
| **15** | 133 | 2452 | 2173 | 2330 | 2088 | 95 | 965 | 466 |
| | 105 | 2067 | 1920 | 1962 | 1831 | 95 | 537 | 290 |
| **16** | 267 | 2494 | 2181 | 2400 | 2106 | 96 | 257 | 219 |
| | 38 | 1166 | 1132 | 1099 | 1076 | 94 | 277 | 233 |
| **17** | 236 | 2282 | 2024 | 2143 | 1917 | 94 | 675 | 419 |
| **18** | 84 | 827 | 892 | 798 | 865 | 96 | 717 | 383 |
| **19** | 218 | 2442 | 2253 | 2358 | 2171 | 97 | 605 | 446 |
| **20** | 182 | 1964 | 1808 | 1876 | 1736 | 96 | 704 | 447 |
| **21** | 567 | 2243 | 2067 | 2127 | 1979 | 95 | 765 | 446 |
| **22** | 128 | 1354 | 1269 | 1288 | 1259 | 95 | 447 | 284 |
| **24** | 313 | 1770 | 1730 | 1683 | 1658 | 95 | 1424 | 706 |
| **25** | 278 | 1452 | 1431 | 1055 | 1091 | 73 | 871 | 959 |
| **26** | 159 | 1753 | 1788 | 1662 | 1603 | 95 | 830 | 490 |
| **27** | 206 | 1683 | 1638 | 1493 | 1529 | 89 | 802 | 434 |
| **28** | 203 | 1520 | 1457 | 1463 | 1411 | 96 | 901 | 458 |
| **29** | 37 | 1367 | 1360 | 1303 | 1297 | 95 | 438 | 279 |
| **30** | 35 | 330 | 371 | 308 | 347 | 93 | 168 | 154 |
| **31** | 17 | 302 | 380 | 265 | 338 | 88 | 178 | 149 |
| **32** | 80 | 1673 | 1649 | 1616 | 1597 | 97 | 957 | 517 |
| **33** | 80 | 567 | 656 | 528 | 636 | 93 | 224 | 148 |
| **34** | 57 | 1445 | 1437 | 1331 | 1348 | 92 | 341 | 301 |
| **35** | 107 | 1201 | 1256 | 1148 | 1199 | 96 | 177 | 193 |
| **36** | 85 | 555 | 609 | 514 | 566 | 93 | 201 | 139 |
| **37** | 87 | 803 | 866 | 776 | 845 | 97 | 242 | 163 |
| **38** | 252 | 1057 | 1123 | 1012 | 1081 | 96 | 767 | 432 |
| **39** | 333 | 1627 | 1466 | 1551 | 1390 | 95 | 1038 | 611 |
| **40** | 177 | 2083 | 1808 | 2023 | 1875 | 97 | 1079 | 447 |

### OPSCC-associated HLA ligands

To identify OPSCC-associated antigens, comparative HLA class I and class II ligandome profiling of the OPSCC cohort was performed against a benign reference dataset. This dataset mainly encompassed HLA ligandome data of a previously reported hematological benign cohort, the benign tissue dataset provided within the HLA Ligand Atlas as well as a newly established tonsillar HLA ligandomeic dataset from five healthy control samples (Table 7).

**Table 7: Overview of HLA ligandomic yields from tonsils. Isolated peptides were analyzed by LC-MS/MS. The HLA class I purity was calculated by the percentage of HLA class I ligands among the total number of HLA class I-presented peptides. T = tonsil.**

| **Sample number** | **Tissue mass [mg]** | **HLA class I** | | | | | **HLA class II** | |
|---|---|---|---|---|---|---|---|---|
| | | **Presented peptides** | **Source proteins** | **Binders** | **Source proteins of binders** | **Purity [%]** | **Presented peptides** | **Source proteins** |
| **T1** | 378 | 2247 | 2097 | 2144 | 2006 | 95 | 1614 | 770 |
| **T2** | 302 | 2728 | 2366 | 2586 | 2272 | 95 | 1026 | 559 |
| **T3** | 210 | 1670 | 1635 | 1602 | 1579 | 96 | 1509 | 649 |
| **T4** | 487 | 2610 | 2350 | 2463 | 2229 | 94 | 2012 | 900 |
| **T5** | 162 | 1685 | 1599 | 1615 | 1542 | 96 | 1044 | 524 |

Together, the benign reference database contained HLA class I ligandome data from 35 hematological and non-hematological tissue types (n = 424 samples) comprising a total of 153,733 unique HLA class I-presented peptides derived from 17,200 different source proteins. Overlap analysis between the OPSCC and the benign reference datasets revealed 5,336 HLA class I ligands presented exclusively on OPSCC samples (Figure 4A). 101 of these tumor-exclusive peptides (TEP) were identified with a prevalence of ≥ 7.5 (≥ 3 samples) among the OPSCC patients. 3,251 TEP were newly identified peptides not present in the inventors' previously published solid tumor immunopeptidomes (ovarian cancer, hepatocellular carcinoma, renal cell carcinoma, glioblastoma).

Regarding the HLA class II ligandomes, the benign reference database was comprised of immunopeptidomic data from 33 hematological and non-hematological tissue types (n = 369 samples) with a total of 156,940 unique HLA class II-presented peptides derived from 16,035 source proteins. Overlap analysis revealed 5,466 OPSCC-exclusive HLA class II-presented peptides (Figure 4B). 82 of these TEP were identified with a prevalence of ≥ 7.5% (≥ 3 samples) among patients. 4,837 TEP were newly identified peptides that were not discovered in the inventors' previously published solid tumor immunopeptidomes (ovarian cancer, renal cell carcinoma, glioblastoma).

### HPV-associated HLA ligands

The present cohort contained 40 tumor samples originating from 22 HPV-positive and 18 HPV-negative patients. In none of the 40 OPSCC immunopeptidomes, HLA class I binders or HLA class II-presented peptides were detected that derived from an HPV source protein or from mutated neoantigens.

However, a supervised principal component analysis (PCA) and corresponding heatmap based on the merged source proteins of HLA class I ligands and of HLA class II-presented peptides resulted in a clear separation of the samples into HPV-positive and HPV-negative tumors (Figure 5). HLA class I binders and HLA class II-presented peptides were identified that were either shared or exclusively presented by HPV-positive or HPV-negative OPSCC samples (Figure 4C-D). Comparative analysis revealed 10,279 HLA class I ligands exclusive for HPV-positive and 5,761 HLA class I ligands exclusive HPV-negative OPSCCs derived from 5,952 and 4,231 source proteins, respectively. Among these, 653 HLA class I ligands exclusive for HPV-positive and 190 HLA class I ligands exclusive for HPV-negative OPSCCs were identified in ≥ 3 samples of the respective subgroup.

7,248 HLA class II-presented peptides exclusive for HPV-positive and 7,720 exclusive for HPV-negative OPSCCs were identified derived from 2,859 and 2,633 source proteins, respectively. Of these, 197 peptides exclusive for HPV-positive and 88 peptides exclusive for HPV-negative OPSCCs were detected in ≥ 3 samples of the respective subgroups.

These results indicate that immunopeptidomes of OPSCCs differ in their composition of antigens depending on the patients' HPV status.

There were no significant differences in the number of total and TEP or total and tumor-exclusive proteins per patient compared by HPV status.

The median number of HLA class I binders per patient and TEP identified was 781.5 (range: 265 - 2,854) and 23.3 (range: 4 - 552), respectively, and the median total number of HLA class I ligand source proteins per patient and tumor-exclusive source proteins was 869.3 (range: 338 - 2,444) and 2 (range: 0 - 11), respectively. Medians with interquartile range are graphed in Figure 6a.

The median total number of HLA class II peptides per patient and TEP identified was 689.5 (range: 168 - 2,086) and 99.5 (range: 20 - 608), respectively, and the median total number of source proteins of HLA class II-presented peptides per patient and tumor-exclusive source proteins was 425.5 (range: 139 - 959) and 4 (range: 0 - 34), respectively. Medians with interquartile range are graphed in Figure 6b.

### Establishment of OPSCC peptide warehouse

Multiobjective optimization employing AVAtar software was used to uncover selections of TEP by HLA allotype with maximal coverage and a minimal number of peptides for the above mentioned 15 HLA allotypes.

Central data from the optimization and selection process are shown in Table 8. In total, 29 TEP were selected for 11 HLA allotypes. For the remaining four allotypes contribution to coverage was negligible due to a low number of patients presenting the same TEP. The resulting selections of TEP for each of the most frequent allotypes are shown in Figure 7 and Table 9. Only 2 HPV-positive patients did not present any of the 29 TEP. The merged selections by HPV status are shown in Figure 8.

**Table 8: Key data of the optimization process using AVAtar. TEP - tumor-exclusive peptide, pt - patient, QC - quality control.**

| **HLA- allele** | **HLA- allotype** | **TEP#** | **TEP# in ≥ 2 pt** | **TEP candidates** | **failed QC** | **TEP selected** | **Coverage of selection** | **Overlap of selection** |
|---|---|---|---|---|---|---|---|---|
| HLA-A | *02:01 | 367 | 57 | 15 | 5 | 7 | 0.762 | 0.667 |
| | *01:01 | 247 | 27 | 8 | 1 | 4 | 0.929 | 0.786 |
| | *24:02 | 121 | 23 | 5 | 2 | 2 | 0.778 | 0.333 |
| | *11:01 | 43 | 2 | 2 | 1 | 0 | n.a. | n.a. |
| | *03:01 | 76 | 3 | 3 | 2 | 0 | n.a. | n.a. |
| HLA-B | *51:01 | 184 | 48 | 3 | 0 | 3 | 0.833 | 0.250 |
| | *07:02 | 95 | 6 | 4 | 2 | 0 | n.a. | n.a. |
| | *08:01 | 128 | 20 | 6 | 2 | 2 | 0.500 | 0.100 |
| | *44:02 | 321 | 45 | 3 | 0 | 1 | 0.500 | 0.000 |
| | *40:01 | 95 | 37 | 6 | 1 | 2 | 1.000 | 0.200 |
| HLA-C | *07:01 | 109 | 6 | 4 | 2 | 2 | 0.385 | 0.000 |
| | *07:02 | 148 | 3 | 1 | 0 | 0 | n.a. | n.a. |
| | *04:01 | 199 | 9 | 2 | 0 | 2 | 0.455 | 0.000 |
| | *03:04 | 264 | 10 | 6 | 3 | 3 | 0.625 | 0.250 |
| | *06:02 | 26 | 2 | 1 | 0 | 1 | 0.429 | 0.000 |

**Table 9: OPSCC warehouse of selected HLA class I ligands (extracts). AC - accession, GN - gene name.**

| **Peptide** | | | | **Source protein** | | |
|---|---|---|---|---|---|---|
| **HLA restriction** | **Sequence** | **Representation frequency in** | | **UniProtKB AC** | **GN** | **Protein name** |
| | | **cohort** | **allo- type- positive sam- ples** | | | |
| **A*02:01** | **ALLGSAFQL** (SEQ ID NO: 19) | 8% | 14% | Q96MG2 | JSRP1 | Junctional sarcoplasmic reticulum protein 1 |
| | **ALTDIVSQV** (SEQ ID NO: 21) | 10% | 18% | Q9NQU5 | PAK6 | Serine/threonine-protein kinase PAK 6 |
| | **GLWEDGRSTLL** (SEQ ID NO: 18) | 8% | 14% | Q96BA8 | CREB3L1 | Cyclic AMP-responsive element-binding protein 3-like protein 1 |
| | **GVLENIFGV** (SEQ ID NO: 15) | 13% | 23% | Q9H6A9 | PCNX3 | Pecanex-like protein 3 |
| | **KLAEISLGV** (SEQ ID NO: 16) | 10% | 18% | P32926 | DSG3 | Desmoglein-3 |
| | **RLDDLKMTV** (SEQ ID NO: 17) | 13% | 23% | Q13753 | LAMC2 | Laminin subunit gamma-2 |
| | **RLLEGEDAHL** (SEQ ID NO: 20) | 15% | 27% | P02533;P0 8779;Q046 95 | KRT14;16 ;17 | Keratin, type I cytoskel-etal 14;16;17 |
| **A*01:01** | **EMEAQNQEY** (SEQ ID NO: 26) | 10% | 29% | P19012 | KRT15 | Keratin, type I cytoskel-etal 15 |
| | **RTDIARTEY** (SEQ ID NO: 27) | 10% | 29% | Q14204 | DYNC1H 1 | Cytoplasmic dynein 1 heavy chain 1 |
| | **RTEFNLNQY** (SEQ ID NO: 25) | 20% | 57% | Q99715 | COL12A1 | Collagen alpha-1 (XII) chain |
| | **YSELASHVVSY** (SEQ ID NO: 28) | 20% | 57% | Q9UMD9 | COL17A1 | Collagen alpha-1 (XVII) chain |
| **C*07:01** | **YTFRYPLSL** (SEQ ID NO: 54) | 8% | 23% | P24347 | MMP11 | Stromelysin-3 |
| | **ARLAFVIVF** (SEQ ID NO: 55) | 5% | 15% | Q5XXA6 | ANO1 | Anoctamin-1 |
| **B*51:01 (B*52:01**) | **DATETTITI** (SEQ ID NO: 39) | 8% | 23% | P02751 | FN1 | Fibronectin |
| | **DQYKFLAV** (SEQ ID NO: 40) | 18% | 54% | Q9NRX3 | NDUFA4L 2 | NADH dehydrogenase [ubiquinone] 1 alpha subcomplex subunit 4-like 2 |
| | **VALPVYLLI** (SEQ ID NO: 41) | 10% | 31% | P57054 | PIGP | Phosphatidylinositol N-acetylglucosaminyl-transferase subunit P |
| **C*04:01** | **SPEDGIHEL** (SEQ ID NO: 57) | 8% | 25% | P02751 | FN1 | Fibronectin |
| | **VFDLVELEVL** (SEQ ID NO: 58) | 5% | 17% | Q8IWT6 | LRRC8A | Leucine-rich repeat-containing protein 8A |
| **B*08:01** | **HGTIKNQL** (SEQ ID NO: 42) | 8% | 30% | Q86XI2 | NCAPG2 | Condensin-2 complex subunit G2 |
| | **VAAPRWVL** (SEQ ID NO: 43) | 8% | 30% | A6NJ16 | IGHV4OR 15-8 | Putative V-set and im-munoglobulin domain-containing-like protein IGHV4OR15-8 |
| **A*24:02 (A*23:01)** | **KYMYFTVVM** (SEQ ID NO: 33) | 18% | 58% | Q8WV24 | PHLDA1 | Pleckstrin homology-like domain family A mem-ber 1 |
| | **NWPSRPYLF** (SEQ ID NO: 34) | 10% | 33% | O43795 | MYO1B | Unconventional myosin-lb |
| **C*03:04** | **SAVKEGTAM** (SEQ ID NO: 60) | 5% | 22% | Q9H694 | BICC1 | Protein bicaudal C hom-olog 1 |
| **B*44:02 (B*44:03)** | **EETNPKGSGW** (SEQ ID NO: 46) | 13% | 64% | Q13835 | PKP1 | Plakophilin-1 |
| **B*40:01** | **REVVDPEVFF** (SEQ ID NO: 49) | 8% | 50% | Q13835 | PKP1 | Plakophilin-1 |
| | **SEPNDVFFKL** (SEQ ID NO: 50) | 8% | 50% | P12111 | COL6A3 | Collagen alpha-3(VI) chain |
| **C*03:04** | **YEEAMKEVL** (SEQ ID NO: 61) | 5% | 22% | Q9P0K7 | RAI14 | Ankycorbin |
| | **YEIDDVERL** (SEQ ID NO: 59) | 8% | 33% | Q9ULI4;Q2 KJY2 | KIF26A;B | Kinesin-like protein KIF26A;B |
| **C*06:02** | **ARLIPIIVL** (SEQ ID NO: 62) | 8% | 43% | B0I1T2 | MYO1G | Unconventional myosin-lg |

Absolute and relative coverage of patients for each of the 11 HLA allotype specific TEP are shown in Figure 9A and 9B, respectively. The selections resulted in a coverage ranging from 100% for HLA-B*40:01 to 38.5% for HLA-C*07:01. The allotype-specific selections covered ≥ 50% of patients with the respective allele for HLA-B*40:01, HLA-A*01:01, HLA-B*51:01, HLA-A*24:02, HLA-A*02:01 HLA-B*08:01 and HLA-B*44:02.

Next, a semi-personalized combination of TEP was selected for every patient based on the individual HLA typing results to mimic a theoretical semi-personalized vaccine composition. These semi-personalized TEP combinations resulted in a median of 9 selected TEP per patient (range: 2 - 19). A median of 2 TEP among the semi-personalized selected TEP were presented in the respective patient's ligandome (range: 0 - 9), resulting in a median coverage of 28.14% of the selected TEP combinations (range: 0 - 100%). Only three patients did not present any of the semi-personalized TEP combinations.

With the described TEP selection, a warehouse was established covering most OPSCC patients and offering potential target peptides for immunotherapeutic approaches in the course of OPSCC treatment.

### 3. Conclusion

Here, the inventors present the first comprehensive analysis of the immunopeptidome in an OPSCC cohort with a representative distribution of HLA allotypes for the German population. Interestingly, HLA-B*51:01 was significantly overrepresented in the OPSCC cohort compared to a reference cohort. The inventors identified OPSCC-exclusive class I and class II peptides in each patient sample including peptides shared by several patients with the respective HLA allele. In total, > 5,000 OPSCC-exclusive class I ligands and 5,468 HLA class II-presented peptides respectively were discovered, some of which were newly identified.

An interesting finding in two patients with cervical cancer treated with adoptive T cell transfer with T cells primed for viral, HPV-specific antigens, challenges the central role of viral antigens even for HPV-associated disease. Interestingly, the main portion of transfused immune cells was not directed against the viral antigens that were used for stimulation and expansion, but against a non-mutated germline associated antigen or a mutation-associated neoantigen, respectively. Thus, viral, mutational and non-mutated cancer antigens may play a role in the recognition of OPSCC.

However in this analysis, none of the TEP was derived from an HPV protein or a predicted, mutated neoantigen. The inventors cannot rule out that such peptides are presented at levels undetectable with the sensitivity of MS applied in this study. However, if they are presented, they are presented at much lower levels than other TEP. No significant differences were found between sample mass, the number of total peptides or TEP presented by HPV status. These results indicate that the failure to detect HPV-peptides cannot be attributed to virally induced reduction of HLA molecules on the tumor. Nevertheless, HPV-associated molecular differences like genetic, epigenetic or transcriptomic alterations were also mirrored in the HLA ligandome resulting in clustering of patient ligandome samples according to HPV status.

Still, HPV-specific T cell immunity has been detected in HPV-associated cancers and is associated with improved prognosis. Also, HPV-specific vaccines have been successful in early clinical trials. Thus, the integration of HPV-specific antigens into a semi-personalized or personalized multiantigen vaccine seems rational due to the high immunogenicity of viral antigens.

The number of individual and shared TEP identified is promising and allows for both, a personalized and a semi-personalized multiantigen vaccination strategy. The strategy described here, is the definition and production of a warehouse of HLA-specific peptide combinations covering a high proportion of untested patients with the respective HLA type based on multiobjective optimization. After immunogenicity testing, from this warehouse, a semi-personalized vaccine will be tested composed of the selection of peptide combinations for the individual patient's HLA type preferably in combination with an HPV-vaccine in HPV-positive patients. This strategy may reach a coverage comparable with a personalized neoepitope vaccine, avoiding the time and cost for individual analysis of the patient's tumor mutanome and manufacturing of a personalized vaccine de *novo.*

Many past and ongoing trials in head and neck cancer focus on neoadjuvant immunotherapy achieving a pathologically confirmed immune response in a high fraction of patients. The semi-personalized strategy makes neoadjvuant immune checkpoint modulation combined with vaccination possible which may further increase response rates. The tumor material harvested during surgery could potentially be used to optimize target antigen selection for an adjuvant phase of immunotherapy. A similar approach has been successfully employed in glioblastoma using a warehouse-based HLA-adapted vaccine followed by personalized vaccination.

In conclusion, the immunopeptidome of OPSCC differs by HPV status although the inventors found no HPV-specific peptides. Instead, a number of TEP, some of which were found repeatedly, was identified and was used to build a peptide warehouse for semi-personalized vaccination as an addition to OPSCC immunotherapy.

## Claims

1. A pharmaceutical composition for the diagnosis and/or prevention and/or treatment of oropharyngeal squamous cell carcinoma (OPSCC) comprising at least one peptide which binds to class II molecules of the major histocompatibility complex (MHC class II molecules) and/or induces T cells cross-reacting with said peptide, and a pharmaceutically acceptable carrier, wherein said at least one peptide comprises an amino acid sequence which is selected from the group consisting of: SEQ ID NO: 1 to SEQ ID NO: 14 and variant sequences thereof which are at least 88% homologous to SEQ ID NO: 1 to SEQ ID NO: 14, wherein said peptide is not a full-length polypeptide.

2. The pharmaceutical composition according to claim 1, wherein it comprises at least 2, preferably at least 3, further preferably at least 4, further preferably at least 5, further preferably at least 6, further preferably at least 7, further preferably at least 8, and highly preferably at least 9 different peptides, each peptide comprising an amino acid sequence which is selected from the group consisting of: SEQ ID NO: 1 to SEQ ID NO: 14 and variant sequences thereof which are at least 88% homologous to SEQ ID NO: 1 to SEQ ID NO: 14, wherein said peptide is not a full-length polypeptide.

3. The pharmaceutical composition according to claim 1 and 2, wherein it comprises different peptides comprising the following amino acid sequences:
IRQFTSSSSIKGSSG (SEQ ID NO: 1)
LTSTSGPGFHLMLPFI (SEQ ID NO: 2)
ELKKKLKEKAKERREKEMLERLEK (SEQ ID NO: 3)
ENDVIISINGQSVVS (SEQ ID NO: 4)
IQDFIEAEDDLSSFR (SEQ ID NO: 5)
PVHWQFGQLDQHPIDG (SEQ ID NO: 6)
FADFTFATGKIIG (SEQ ID NO: 7)
IKNIISEYKSAIQSQKRRRPRYRKR (SEQ ID NO: 8)
VDEEEVKFPGTNFDE (SEQ ID NO: 9).

4. The pharmaceutical composition according to any of the preceding claims, wherein it comprises at least one additional peptide which binds to class I molecules of the major histocompatibility complex (MHC class I molecules) and/or induces T cells cross-reacting with said peptide, wherein said at least one additional peptide comprises an amino acid sequence which is selected from the group consisting of: SEQ ID NO: 15 to SEQ ID NO: 62 and variant sequences thereof which are at least 88% homologous to SEQ ID NO: 15 to SEQ ID NO: 62, wherein said peptide is not a full-length polypeptide.

5. The pharmaceutical composition according to claim 4, wherein the at least one additional peptide is selected depending on the MHC class I allotype of the individual to be treated.

6. The pharmaceutical composition according to claim 5, wherein the at least one additional peptide is selected as follows:
| MHC class I allotype | Amino acid sequence of peptide |
|---|---|
| A*02:01 | SEQ ID NO: 15 to SEQ ID NO: 24 |
| A*01:01 | SEQ ID NO: 25 to SEQ ID NO: 31 |
| A*03:01 | SEQ ID NO: 32 |
| A*24:02 | SEQ ID NO: 33 to SEQ ID NO: 35 |
| A*11:01 | SEQ ID NO: 36 |
| B*07:02 | SEQ ID NO: 37 to SEQ ID NO: 38 |
| B*51:01 | SEQ ID NO: 39 to SEQ ID NO: 41 |
| B*08:01 | SEQ ID NO: 42 to SEQ ID NO: 45 |
| B*44:02 | SEQ ID NO: 46 to SEQ ID NO: 48 |
| B*40:01 | SEQ ID NO: 49 to SEQ ID NO: 53 |
| C*07:01 | SEQ ID NO: 54 to SEQ ID NO: 55 |
| C*07:02 | SEQ ID NO: 56 |
| C*04:01 | SEQ ID NO: 57 to SEQ ID NO: 58 |
| C*03:04 | SEQ ID NO: 59 to SEQ ID NO: 61 |
| A*02:01 | SEQ ID NO: 62 |
, wherein the amino acid sequences of each MHC class I allotype group includes variant sequences which are at least 88% homologous.

7. The pharmaceutical composition according to any of claims 4 to 6, wherein it comprises at least 2, preferably at least 3, further preferably at least 4, further preferably at least 5, and highly preferably at least 6 different additional peptides, each additional peptide comprising an amino acid sequence which is selected from the group consisting of: SEQ ID NO: 15 to SEQ ID NO: 62 and variant sequences thereof which are at least 88% homologous to SEQ ID NO: 15 to SEQ ID NO: 62, wherein said additional peptide is not a full-length polypeptide.

8. The pharmaceutical composition according to any of the preceding claims which is a vaccine.

9. A peptide comprising an amino acid sequence selected from the group consisting of SEQ ID NO: 1 to SEQ ID NO: 62, and variant sequences thereof which are at least 88% homologous to SEQ ID NO: 1 to SEQ ID NO: 62, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide,
preferably
said peptide has the ability to bind to an MHC class I or II molecule, and wherein said peptide, when bound to said MHC, is capable of being recognized by CD4 and/or CD8 T cells,
further preferably
the amino acid sequence thereof comprises a continuous stretch of amino acids according to any one of SEQ ID NO: 1 to SEQ ID NO: 62.

10. A nucleic acid, encoding for a peptide or variant thereof according to claim 9, optionally linked to a heterologous promoter sequence, or an expression vector expressing said nucleic acid.

11. A recombinant host cell comprising the peptide according to claim 9 or the nucleic acid or the expression vector according to claim 10, wherein said host cell preferably is selected from an antigen presenting cell, such as a dendritic cell, a T cell or an NK cell.

12. An *in vitro* method for producing activated T lymphocytes, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to claim 9.

13. An activated T lymphocyte, produced by the method according to claim 16, that selectively recognizes a cell which presents a polypeptide comprising an amino acid sequence given in claim 9.

14. A kit comprising:
(a) a container comprising the pharmaceutical composition according to any one of claims 1-8, the peptide(s) according to claim 9, the nucleic acid or the expression vector according to claim 10, the recombinant host cell according to claim 11 or the activated T lymphocyte according to claim 13, in solution or in lyophilized formulation;
(b) optionally, a second container containing a diluent or reconstituting solution for the lyophilized formulation;
(c) optionally, instructions for (i) use of the solution or (ii) reconstitution and/or use of the lyophilized formulation.

15. A method for producing a personalized anti-cancer vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or over-presentation in tumors as compared to normal tissues;
c) selecting at least one peptide from the warehouse that matched a TUMAP identified in said patient; and
d) formulating the personalized vaccine based on step c),
wherein said warehouse comprises a plurality of peptides and/or variant sequences according to claim 9.
